# EUROPEAN PATENT APPLICATION

(11) **EP 4 850 685 A2**
(43) Date of publication of application: **09.12.2026**
(21) Application number: 26188824.2
(22) Date of filing: 13.07.2020
(51) Int. Cl.: A61K 39/00

(54) **ANTI-TAU ANTIBODY AND USE OF SAME**

(30) Priority: 15.07.2019 KR 20190085233
(62) Document number(s) of the earlier application(s) in accordance with Art. 76 EPC: 20841174.4 / 4 001 305
(71) Applicant: Adel Inc., Seoul 05505 (KR)
(72) Inventor: YOON, Seung-Yong, 05505 Seoul (KR)
(74) Representative: Potter Clarkson

(57) **Abstract**

The present invention relates to an anti-tau antibody that specifically binds to tau proteins and uses thereof. The anti-tau antibody according to the present invention can specifically bind to a tau protein, in which the 280^{th} lysine is acetylated, and it can inhibit aggregation of abnormal tau proteins. In addition, in a case where the anti-tau antibody according to the present invention is administered to a dementia-induced animal model, the anti-tau antibody can improve the motor function and cognitive function of the animal model. Accordingly, the anti-tau antibody according to the present invention can be effectively used for preventing or treating a degenerative neurological disease.

## Description

### Technical Field

The present invention relates to an anti-tau antibody that specifically binds to tau protein, and a use thereof.

### Background Art

Alzheimer's disease, which accounts for about 50% of onset forms of dementia, is a degenerative cranial nerve disease with an increased onset rate since the age of 65, and it is rapidly increasing all over the world as the population ages.

It has been believed that the cause for onset of Alzheimer's disease is mainly attributed to accumulation of β-amyloid, hyperphosphorylation of tau protein, or increased β-amyloid production caused by presenilin 1. Among these, nerve cell toxicity caused by accumulation of β-amyloid has been thought to be a major cause for onset of Alzheimer's dementia (Hardy AJ et al., Science, 256, 184-185, 1992). However, as a phase III trial for solanezumab which was an Eli Lilly and Company's new drug candidate for Alzheimer's disease failed, it has been found that nerve cell toxicity caused by accumulation of β-amyloid is not remarkable. Therefore, focuses are on the idea that abnormal hyperphosphorylation of tau protein acts as a major cause for onset of Alzheimer's disease.

Tau protein is a protein that stabilizes the microtubule which is a protein that transports a cellular material. The tau protein exists in six isoforms in the human body and is abundant in neurons of the central nervous system. In addition, in a case where a mutation occurs in the tau protein, it is known that the tau protein is hyperphosphorylated, which allows neurofibrillary tangles (NFTs) to be abnormally accumulated in nerve cells, thereby causing a degenerative neurological disease such as dementia and Parkinson's disease (Dong Hee Choi et al., Brain & NeuroRehabilitation, 4, 21-29, 2011).

However, the human tau protein consisting of 441 amino acids has a wide variety of locations where post-translational modification may occur, which makes it difficult to find, in the tau protein, a target portion that exhibits a preventive or therapeutic effect on dementia. Due to such various modifications, there are also difficulties in developing a therapeutic agent.

### Disclosure of Invention

### Technical Problem

Accordingly, the present inventors have studied to develop an effective therapeutic agent for a degenerative neurological disease. As a result, the present inventors have found that an antibody, which specifically binds to a fragment of the tau protein in which the 280^{th} lysine is acetylated, decreases aggregation of tau proteins. In addition, the present inventors have found that in experiments using a dementia-induced animal model, the animal model into which the antibody has been administered exhibits improved motor function and cognitive function.

### Solution to Problem

In order to solve the above problems, in an aspect of the present invention, there is provided an anti-tau antibody or an antigen-binding fragment thereof, which comprises (i) a heavy chain variable region (VH) that includes a heavy chain CDR1 having the amino acid sequence represented by SEQ ID NO: 1, a heavy chain CDR2 having the amino acid sequence represented by SEQ ID NO: 2, and a heavy chain CDR3 having the amino acid sequence represented by SEQ ID NO: 3 and (ii) a light chain variable region (VL) that includes a light chain CDR1 having the amino acid sequence represented by SEQ ID NO: 4, a light chain CDR2 having the amino acid sequence represented by SEQ ID NO: 5, and a light chain CDR3 having the amino acid sequence represented by SEQ ID NO: 6.

In another aspect of the present invention, there is provided a polynucleotide that encodes the anti-tau antibody or antigen-binding fragment thereof, or the heavy chain variable region and/or light chain variable region of the anti-tau antibody.

In another aspect of the present invention, there is provided an expression vector comprising the polynucleotide.

In yet another aspect of the present invention, there is provided a host cell into which the expression vector is introduced.

In still yet another aspect of the present invention, there is provided a method for producing an anti-tau antibody or an antigen-binding fragment thereof, the method comprising a step of culturing the host cell.

In still yet another aspect of the present invention, there is provided a hybridoma having accession number KCTC 14155BP.

In still yet another aspect of the present invention, there is provided a pharmaceutical composition for preventing or treating a degenerative neurological disease, the pharmaceutical composition comprising, as an active ingredient, the anti-tau antibody or antigen-binding fragment thereof.

In still yet another aspect of the present invention, there is provided a composition for diagnosing a degenerative neurological disease, the composition comprising the anti-tau antibody or antigen-binding fragment thereof.

In still yet another aspect of the present invention, there is provided a kit for preventing, treating, or diagnosing a degenerative neurological disease, the kit comprising the antibody or antigen-binding fragment thereof, the polynucleotide, the expression vector, or the host cell.

### Advantageous Effects of Invention

The anti-tau antibody according to the present invention can specifically bind to tau protein, in which the 280^{th} lysine is acetylated, and inhibit aggregation of abnormal tau proteins. In addition, in a case where the anti-tau antibody according to the present invention is administered to a dementia-induced animal model, the anti-tau antibody can improve the motor function and cognitive function of the animal model. Accordingly, the anti-tau antibody according to the present invention can be advantageously used for preventing or treating a degenerative neurological disease.

### Brief Description of Drawings

Fig. 1 schematically illustrates a tau protein fragment K280-ac prepared in an embodiment of the present invention.
Fig. 2 is a schematic diagram of a expression vector pET-21b-Tau (K18) used in an embodiment of the present invention.
Fig. 3 illustrates the result of a Western blot test performed to identify binding between an antibody and the protein fragment K280-ac in order to select a monoclonal antibody that specifically binds to the protein fragment K280-ac.
Fig. 4 illustrates the result of an ELISA test performed to identify binding between an antibody and the protein fragment K280-ac in order to select a monoclonal antibody that specifically binds to the protein fragment K280-ac.
Fig. 5 illustrates the result of a test performed to identify whether a selected antibody ADEL-Y01 binds to an acetylated wild type tau protein (Tau) and a tau protein (Tau K280A) having the 280^{th} amino acid lysine substituted with alanine.
Fig. 6 illustrates the result of a test performed to identify whether an acetylated wild type protein and a non-acetylated wild type tau protein specifically bind to ADEL-Y01h_v01 antibody. Fig. 7 illustrates dissociation constant (Kd) values measured using Octet^{®} K2 system to identify an affinity between an ADEL-Y01m antibody and the protein fragment K280-ac.
Fig. 8 illustrates the result of an ELISA test performed to identify binding between ADEL-Y01h antibodies (v01 to v12) and the protein fragment K280-ac.
Fig. 9 illustrates expression levels and patterns of a tau protein (Tau5) and an acetylated tau protein (Tau-acK208) in brain tissues of normal mice (wild type) and dementia mice (Tau P301L) which are 4-month-old or 12-month-old.
Fig. 10 illustrates an experimental schedule for identifying a behavior-improving effect achieved upon intracerebroventricular administration of the ADEL-Y01m antibody using a dementia-induced mouse model.
Fig. 11 illustrates the result of a nesting building test (*** p<0.001) in which the ADEL-Y01m antibody is intracerebroventricularly administered into a dementia-induced mouse model.
Fig. 12 illustrates the result of a Y-maze test (* p<0.05) in which the ADEL-Y01m antibody is intracerebroventricularly administered into a dementia-induced mouse model.
Fig. 13 illustrates the result of a water maze test in which the ADEL-Y01m antibody is intracerebroventricularly administered into a dementia-induced mouse model.
Fig. 14 illustrates the time spent staying in each zone in a water maze test (** p<0.01) in which the ADEL-Y01m antibody is intracerebroventricularly administered into a dementia-induced mouse mode.
Fig. 15 illustrates the result of a grip strength test (* p<0.05, ** p<0.01) in which the ADEL-Y01m antibody is intracerebroventricularly administered into a dementia-induced mouse model.
Fig. 16 illustrates an experimental schedule for identifying a behavior-improving effect achieved upon intraperitoneal administration of the ADEL-Y0 1m antibody using a dementia-induced mouse model.
Fig. 17 illustrates the result of a nesting building test (* p<0.05, ** p<0.01) in which the ADEL-Y01m antibody is intraperitoneally administered into a dementia-induced mouse model.
Fig. 18 illustrates the result of a Y-maze test (* p<0.05) in which the ADEL-Y01m antibody is intraperitoneally administered into a dementia-induced mouse model.
Fig. 19 illustrates the result of a water maze test in which the ADEL-Y01m antibody is intraperitoneally administered into a dementia-induced mouse model.
Fig. 20 illustrates the time spent staying in each zone in a water maze test (* p<0.05) in which the ADEL-Y01m antibody is intraperitoneally administered into a dementia-induced mouse model before the water maze test is performed.
Fig. 21 illustrates the result of immunoprecipitation and Western blot tests performed to identify binding between the ADEL-Y01m antibody and an acetylated tau protein (Tau-acK280) expressed in the cerebral cortices and the hippocampal tissues of normal mice (wild type) or a dementia-induced mouse model (Tau P301L).
Fig. 22 illustrates photographs taken after brain tissues of normal elderly and brain tissues of patients with Alzheimer's disease are subjected to immunohistochemical staining using the ADEL-Y01m antibody.
Fig. 23A illustrates the result of a test performed to identify expression levels of the entire tau protein (Tau5), an acetylated tau protein (Tau-acK280), and amyloid-β in brain tissues of normal elderly or brain tissues of patients with Alzheimer's disease.
Fig. 23B illustrates the result of a test performed to identify binding between the ADEL-Y01m antibody and an acetylated tau protein expressed in brain tissues of normal elderly or brain tissues of patients with Alzheimer's disease (* p<0.05).
Fig. 24 illustrates the result of a test performed to identify expression levels of the entire tau protein (Tau5), an acetylated tau protein (Tau-acK280), and a phosphorylated tau protein (pSer396) in brain tissues of normal mice, a dementia-induced mouse model, and a dementia-induced mouse model into which the ADEL-Y01m antibody is intracerebroventricularly administered.
Fig. 25 illustrates expression levels of the entire tau protein (Tau5) in the brain tissues of normal mice, a dementia-induced mouse model, and a dementia-induced mouse model into which the ADEL-Y01m antibody is intracerebroventricularly administered (* p<0.05, *** p<0.001).
Fig. 26 illustrates expression levels of the phosphorylated tau protein (pSer396) in the brain tissues of normal mice, a dementia-induced mouse model, and a dementia-induced mouse model into which the ADEL-YOlm antibody is intracerebroventricularly administered (** p<0.01).
Fig. 27 illustrates expression levels of the acetylated tau protein (Tau-acK280) in the brain tissues of normal mice, a dementia-induced mouse model, and a dementia-induced mouse model into which the ADEL-YOlm antibody is intracerebroventricularly administered.
Fig. 28 illustrates photographs showing brain tissues immunostained with the ADEL-Y01m antibody, wherein the brain tissues are obtained from normal mice, a dementia-induced mouse model, and a dementia-induced mouse model (Tau-P301L-ADEL-Y01m) into which the ADEL-Y01m antibody is intracerebroventricularly administered.
Fig. 29 illustrates expression levels of AT8 and phosphorylated tau protein (pT231) in brain tissues of normal mice, a dementia-induced mouse model, and a dementia-induced mouse model (Tau-P301L-ADEL-Y01m) into which the ADEL-Y01m antibody is intracerebroventricularly administered (* p<0.05).
Fig. 30 illustrates expression levels of the entire tau protein (Tau5), an acetylated tau protein (Tau-acK280), and phosphorylated tau protein (pT231) in brain tissues of normal mice, a dementia-induced mouse model, and a dementia-induced mouse model (Tau-P301L-ADEL-Y01m) into which the ADEL-Y01m antibody is intraperitoneally administered.
Fig. 31 illustrates expression levels of the entire tau protein (Tau5) in the brain tissues of normal mice, a dementia-induced mouse model, and a dementia-induced mouse model into which the ADEL-Y01m antibody is intraperitoneally administered (* p<0.05).
Fig. 32 illustrates expression levels of the acetylated tau protein (Tau-acK280) in the brain tissues of normal mice, a dementia-induced mouse model, and a dementia-induced mouse model into which the ADEL-Y01m antibody is intraperitoneally administered (* p<0.05).
Fig. 33 illustrates expression levels of the phosphorylated tau protein (pT231) in the brain tissues of normal mice, a dementia-induced mouse model, and a dementia-induced mouse model into which the ADEL-Y01m antibody is intraperitoneally administered (* p<0.05).
Fig. 34 illustrates expression levels of synaptic-related proteins (PSD95 and synapsin-1) in brain tissues of normal mice, a dementia-induced mouse model, and a dementia-induced mouse model into which the ADEL-Y01m antibody is intracerebroventricularly administered.
Fig. 35 illustrates expression levels of PSD95 in the brain tissues of normal mice, a dementia-induced mouse model, a dementia-induced mouse model into which the ADEL-Y01m antibody is intracerebroventricularly administered (* p<0.05).
Fig. 36 illustrates expression levels of synapsin-1 in the brain tissues of normal mice, a dementia-induced mouse model, a dementia-induced mouse model into which the ADEL-Y01m antibody is intracerebroventricularly administered (* p<0.05).
Fig. 37 illustrates expression levels of synaptic-related proteins (PSD95 and synapsin-1) in brain tissues of normal mice, a dementia-induced mouse model, and a dementia-induced mouse model into which the ADEL-Y01m antibody is intraperitoneally administered.
Fig. 38 illustrates expression levels of PSD95 in the brain tissues of normal mice, a dementia-induced mouse model, and a dementia-induced mouse model into which the ADEL-Y01m antibody is intraperitoneally administered (* p<0.05).
Fig. 39 illustrates expression levels of synapsin-1 in the brain tissues of normal mice, a dementia-induced mouse model, and a dementia-induced mouse model into which the ADEL-Y01m antibody is intraperitoneally administered (* p<0.05).
Fig. 40 illustrates the result of a test performed to identify whether the ADEL-Y01m antibody penetrates brain tissues of normal mice, a dementia-induced mouse model, and a dementia-induced mouse model into which the ADEL-Y01m antibody is intraperitoneally administered, and whether the antibody is bound to tau protein.
Fig. 41 illustrates an experimental schedule for identifying a behavior-improving effect achieved upon intracerebroventricular administration of the ADEL-Y01m antibody using an aged dementia-induced mouse model.
Fig. 42 illustrates the result of a nesting building test (* p<0.05) in which the ADEL-Y01m antibody is intracerebroventricularly administered into an aged dementia-induced mouse model.
Fig. 43 illustrates the result of a Y-maze test (* p<0.05) in which the ADEL-Y01m antibody is intracerebroventricularly administered into an aged dementia-induced mouse model.
Fig. 44 illustrates the time spent staying in each zone in a water maze test (* p<0.05) in which the ADEL-Y01m antibody is intracerebroventricularly administered into an aged dementia-induced mouse model.
Fig. 45 illustrates expression levels of the entire tau protein (Tau5), an acetylated tau protein (Tau-acK280), and a phosphorylated tau protein (pT231) in brain tissues of normal mice, an aged dementia-induced mouse model, and an aged dementia-induced mouse model into which the ADEL-Y01m antibody is intraperitoneally administered.
Fig. 46 illustrates expression levels of the entire tau protein (Tau5) in the brain tissues of normal mice, an aged dementia-induced mouse model, and an aged dementia-induced mouse model into which the ADEL-Y01m antibody is intraperitoneally administered (* p<0.05, ** p<0.01).
Fig. 47 illustrates expression levels of the acetylated tau protein (Tau-acK280) in the brain tissues of normal mice, an aged dementia-induced mouse model, and an aged dementia-induced mouse model into which the ADEL-Y01m antibody is intraperitoneally administered (** p<0.01).
Fig. 48 illustrates expression levels of the entire phosphorylated tau protein (pT231) in the brain tissues of normal mice, an aged dementia-induced mouse model, and an aged dementia-induced mouse model into which the ADEL-Y01m antibody is intraperitoneally administered (* p<0.05).
Fig. 49 illustrates the result of a test performed to identify, for each fraction, insoluble tau aggregates in cerebral cortices of normal mice, an aged dementia-induced mouse model, and an aged dementia-induced mouse model into which the ADEL-Y01m antibody is intraperitoneally administered.
Fig. 50 illustrates expression levels of hemagglutinin (HA), the entire tau protein (Tau5), and an acetylated tau protein (Tau-acK280) which are identified by a Western blot test performed after subjecting donor cells treated with an acetylated tau protein to lysis.
Fig. 51 illustrates expression levels of hemagglutinin (HA), the entire tau protein (Tau5), and an acetylated tau protein (Tau-acK280) which are identified by Western blot test performed after obtaining media of donor cells treated with an acetylated tau protein.
Fig. 52 illustrates intracellular tau protein aggregates identified by a Western blot test performed after applying a culture of donor cells treated with an acetylated tau protein to nerve cells and subjecting the nerve cells to lysis after 1 hour or 20 hours.
Fig. 53 illustrates intracellular tau protein aggregates identified by Western blot and immunoprecipitation tests after applying a culture of donor cells treated with an acetylated tau protein to nerve cells and subjecting the nerve cells to lysis after 1 hour or 20 hours.
Fig. 54 illustrates tau protein seeding inhibitory effect (** p<0.01, *** p<0.001) identified by FRET performed after subjecting Tau-RD-P301S FRET biosensor cells to treatment with a protein fragment K280-ac or an aggregate thereof and the ADEL-Y01h01_v01 antibody.
Fig. 55 illustrates tau protein seeding inhibitory effect (** p<0.01) identified by fluorescence resonance energy transfer (FRET) performed after subjecting Tau-RD-P301S FRET biosensor cells to treatment with a Sarkosyl insoluble fraction from patients with Alzheimer's disease and with the ADEL-Y01h01_v01 antibody.
Fig. 56 illustrates photographs showing brain tissues immunostained with an anti-AT8 antibody, wherein the brain tissues are extracted after the Sarkosyl insoluble fraction from patients with Alzheimer's disease is administered into CA1 layer in the left hippocampus of Tau-P301L dementia mice (Ipsi: ipsilateral, Contra: contralateral).
Fig. 57 illustrates photographs showing brain tissues immunostained with an anti-AT8 antibody, wherein the brain tissues are extracted after the Sarkosyl insoluble fraction from patients with Alzheimer's disease is administered into CA1 layer in the left hippocampus of Tau-P301L dementia mice (DG: dentate gyrus, EC: entorhinal cortex).
Figs. 58 and 59 are graphs showing the extent that brain tissues are immunostained with an anti-AT8 antibody, wherein the brain tissues are extracted after the Sarkosyl insoluble fraction from patients with Alzheimer's disease is administered into CA1 layer in the left hippocampus of Tau-P301L dementia mice (* p<0.05, ** p<0.01). Fig. 60 illustrates the result of a Thioflavin-T test performed to identify changes in aggregation of the acetylated tau proteins after treating an acetylated tau protein with IgG or ADEL-Y01m antibody.
Fig. 61 illustrates the result of a test performed to identify changes in the amount of aggregated acetylated tau proteins (Tau-acK280) after treating a tau protein (K18), an acetylated tau protein (K18 + P300), and an aggregated acetylated tau protein (K18 + P300 + heparin) with the ADEL-Y01m antibody.
Fig. 62 is a graph showing the activity of lactate dehydrogenase (LDH) that is contained in a supernatant obtained after mouse-derived nerve cells treated with IgG or ADEL-Y01m antibody are treated with acetylated tau protein aggregates (*** p<0.001).
Fig. 63 is a graph showing the viability of nerve cells that are obtained after mouse-derived nerve cells treated with IgG or ADEL-Y01m antibody are treated with acetylated tau protein aggregates (*p<0.05, ***p<0.001).
Fig. 64 is graphs showing the fluorescence of HiLyte^{™} Fluor 488 of a microglia that is obtained after mouse-derived microglia treated with IgG or ADEL-YOlm antibody is treated with HiLyte^{™} Fluor 488-labeled acetylated tau protein aggregates.

### Detailed Description of Invention

Hereinafter, the present invention will be described in detail.

In an aspect of the present invention, there is provided an anti-tau antibody or an antigen-binding fragment thereof, which comprises a heavy chain variable region (VH) that includes a heavy chain CDR1 having the amino acid sequence represented by SEQ ID NO: 1, a heavy chain CDR2 having the amino acid sequence represented by SEQ ID NO: 2, and a heavy chain CDR3 having the amino acid sequence represented by SEQ ID NO: 3 and a light chain variable region (VL) that includes a light chain CDR1 having the amino acid sequence represented by SEQ ID NO: 4, a light chain CDR2 having the amino acid sequence represented by SEQ ID NO: 5, and a light chain CDR3 having the amino acid sequence represented by SEQ ID NO: 6.

The term "antibody" as used herein means an immune protein that can bind to an antigen, thereby interfering with an action of the antigen or eliminating the antigen. There are five types of antibodies: IgM, IgD, IgG, IgA, and IgE, and these antibodies contain heavy chains produced by heavy chain constant region genes, µ, δ, γ, α, and ε, respectively. In techniques using antibodies, IgG is mainly used. IgG has four isotypes: IgG1, IgG2, IgG3, and IgG4, and their structural and functional characteristics may be different.

The IgG forms a Y-shaped stable structure (molecular weight: about 150 kDa) made up of two heavy chain (about 50 kDa) proteins and two light chain (about 25 kDa) proteins. In antibodies, light and heavy chains consist of a variable region of which an amino acid sequence differs from one antibody to another, and a constant region of which an amino acid sequence is the same among antibodies. There are CH1, H (hinge), CH2, and CH3 domains in the heavy chain constant region. Each of the domains is composed of two β-sheets, and the domains are connected to each other via an intramolecular disulfide bond. Two variable regions of heavy and light chains are combined to form an antigen binding site. The antigen binding site exists on each of the two Y-shaped arms. A portion capable of binding to an antigen is called Fab (antibody binding fragment), and a portion that does not bind to an antigen is called Fc (crystallizable fragment). Fab and Fc are connected to each other via a flexible hinge region.

The term "CDR" as used herein means a hypervariable region that is a site which is in heavy chain and light chain variable regions of an antibody and of which an amino acid sequence differs from one antibody to another, wherein the site binds to an antigen. In a three dimensional structure of an antibody, CDR is formed as a loop on a surface of the antibody, and under the loop, a framework region (FR) exists for structurally supporting the CDR. There are three loop structures in the heavy chain, there are three loop structures in the light chain, and these six regional loop structures are combined together to directly contact an antigen.

The heavy chain variable region may include any one amino acid sequence selected from the group consisting of SEQ ID NOs: 7 to 11, and the light chain variable region may include any one amino acid sequence selected from the group consisting of SEQ ID NOs: 12 to 15.

In addition, the anti-tau antibody or antigen-binding fragment thereof may be any one selected from the following group:
an anti-tau antibody or antigen-binding fragment thereof which comprises a heavy chain variable region having the amino acid sequence represented by SEQ ID NO: 7 and a light chain variable region having the amino acid sequence represented by SEQ ID NO: 12;
an anti-tau antibody or antigen-binding fragment thereof which comprises a heavy chain variable region having the amino acid sequence represented by SEQ ID NO: 8 and a light chain variable region having the amino acid sequence represented by SEQ ID NO: 13;
an anti-tau antibody or antigen-binding fragment thereof which comprises a heavy chain variable region having the amino acid sequence represented by SEQ ID NO: 8 and a light chain variable region having the amino acid sequence represented by SEQ ID NO: 14;
an anti-tau antibody or antigen-binding fragment thereof which comprises a heavy chain variable region having the amino acid sequence represented by SEQ ID NO: 8 and a light chain variable region having the amino acid sequence represented by SEQ ID NO: 15;
an anti-tau antibody or antigen-binding fragment thereof which comprises a heavy chain variable region having the amino acid sequence represented by SEQ ID NO: 9 and a light chain variable region having the amino acid sequence represented by SEQ ID NO: 13;
an anti-tau antibody or antigen-binding fragment thereof which comprises a heavy chain variable region having the amino acid sequence represented by SEQ ID NO: 9 and a light chain variable region having the amino acid sequence represented by SEQ ID NO: 14;
an anti-tau antibody or antigen-binding fragment thereof which comprises a heavy chain variable region having the amino acid sequence represented by SEQ ID NO: 9 and a light chain variable region having the amino acid sequence represented by SEQ ID NO: 15;
an anti-tau antibody or antigen-binding fragment thereof which comprises a heavy chain variable region having the amino acid sequence represented by SEQ ID NO: 10 and a light chain variable region having the amino acid sequence represented by SEQ ID NO: 13;
an anti-tau antibody or antigen-binding fragment thereof which comprises a heavy chain variable region having the amino acid sequence represented by SEQ ID NO: 10 and a light chain variable region having the amino acid sequence represented by SEQ ID NO: 14;
an anti-tau antibody or antigen-binding fragment thereof which comprises a heavy chain variable region having the amino acid sequence represented by SEQ ID NO: 10 and a light chain variable region having the amino acid sequence represented by SEQ ID NO: 15;
an anti-tau antibody or antigen-binding fragment thereof which comprises a heavy chain variable region having the amino acid sequence represented by SEQ ID NO: 11 and a light chain variable region having the amino acid sequence represented by SEQ ID NO: 13;
an anti-tau antibody or antigen-binding fragment thereof which comprises a heavy chain variable region having the amino acid sequence represented by SEQ ID NO: 11 and a light chain variable region having the amino acid sequence represented by SEQ ID NO: 14; and
an anti-tau antibody or antigen-binding fragment thereof which comprises a heavy chain variable region having the amino acid sequence represented by SEQ ID NO: 11 and a light chain variable region having the amino acid sequence represented by SEQ ID NO: 15.

In a case where CDR1, CDR2, and CDR3 of the light chain variable region or heavy chain variable region are the same, it is possible to modify a framework portion thereof. In particular, the amino acid sequence of a part of the framework portion may be modified to prepare a humanized antibody.

Amino acid sequences and SEQ ID NOs. of the anti-tau antibodies according to the examples of the present invention are summarized in Tables 1 and 2 below.

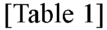

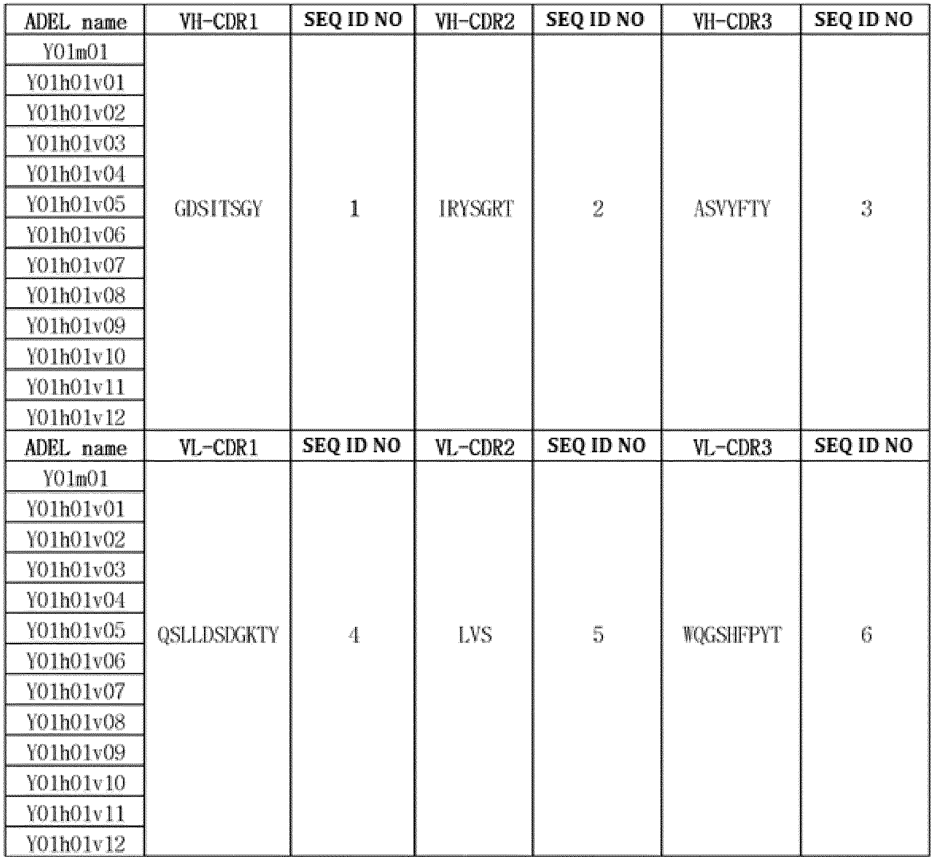

**[Table 2]**

| **ADEL name** | **VH amino acid sequence** | **SEQ ID NO** | **VL amino acid sequence** | **SEQ ID NO** |
|---|---|---|---|---|
| Y01m01 | EVQLEESGPSLVKPSQTLSLTCSV TGDSITSGYWNWIRKFPGNKLEY MGYIRYSGRTYYNPSLKSRISITR DTSKNQFYLQLISVTTEDTATYY CASVYFTYWGQGTLVTVSS | 7 | DVLMTQTPLTLSVTIGQPASI SCKSSQSLLDSDGKTYLNW LLQRPGQSPKRLISLVSKLDS GVPDRFTGSGSGTDFTLKIS RVEAEDLGVYYCWQGSHFP YTFGGGTKLEIK | 12 |
| Y01h01v01 | EVQLQESGPGLVKPSQTLSLTCT VSGDSITSGYWNWIRQPPGKGLE YIGYIRYSGRTYYNPSLKSRVTIS RDTSKNQFSLKLSSVTAADTAVY YCASVYFTYWGQGTLVTVSS | 8 | DVVMTQSPLSLPVTLGQPAS ISCKSSQSLLDSDGKTYLNW FQQRPGQSPKRLISLVSKLD SGVPDRFSGSGSGTDFTLKIS RVEAEDVGVYYCWQGSHFP | 13 |
| | | | YTFGQGTKLEIK | |
| Y01h01v02 | EVQLQESGPGLVKPSQTLSLTCT VSGDSITSGYWNWIRQPPGKGLE YIGYIRYSGRTYYNPSLKSRVTIS RDTSKNQFSLKLSSVTAADTAVY YCASVYFTYWGQGTLVTVSS | 8 | DVVMTQSPDSLAVSLGERA TINCKSSQSLLDSDGKTYLN WYQQKPGQSPKRLISLVSKL DSGVPDRFSGSGSGTDFTLTI SSLQAEDVAVYYCWQGSHF PYTFGQGTKLEIK | 14 |
| Y01h01v03 | EVQLQESGPGLVKPSQTLSLTCT VSGDSITSGYWNWIRQPPGKGLE YIGYIRYSGRTYYNPSLKSRVTIS RDTSKNQFSLKLSSVTAADTAVY YCASVYFTYWGQGTLVTVSS | 8 | EVVLTQSPGTLSLSPGERAT LSCKSSQSLLDSDGKTYLN WYQQKPGQSPKRLISLVSKL DSGIPDRFSGSGSGTDFTLTI SRLEPEDFAVYYCWQGSHFP YTFGQGTKLEIK | 15 |
| Y01h01v04 | EVQLQQSGPGLVKPSQTLSLTCA VSGDSITSGYWNWIRQSPSRGLE WLGYIRYSGRTYYNPSLKSRITIN RDTSKNQFSLQLNSVTPEDTAVY YCASVYFTYWGQGTLVTVSS | 9 | DVVMTQSPLSLPVTLGQPAS ISCKSSQSLLDSDGKTYLNW FQQRPGQSPKRLISLVSKLD SGVPDRFSGSGSGTDFTLKIS RVEAEDVGVYYCWQGSHFP YTFGQGTKLEIK | 13 |
| Y01h01v05 | EVQLQQSGPGLVKPSQTLSLTCA VSGDSITSGYWNWIRQSPSRGLE WLGYIRYSGRTYYNPSLKSRITIN RDTSKNQFSLQLNSVTPEDTAVY YCASVYFTYWGQGTLVTVSS | 9 | DVVMTQSPDSLAVSLGERA TINCKSSQSLLDSDGKTYLN WYQQKPGQSPKRLISLVSKL DSGVPDRFSGSGSGTDFTLTI SSLQAEDVAVYYCWQGSHF PYTFGQGTKLEIK | 14 |
| Y01h01v06 | EVQLQQSGPGLVKPSQTLSLTCA VSGDSITSGYWNWIRQSPSRGLE WLGYIRYSGRTYYNPSLKSRITIN RDTSKNQFSLQLNSVTPEDTAVY YCASVYFTYWGQGTLVTVSS | 9 | EVVLTQSPGTLSLSPGERAT LSCKSSQSLLDSDGKTYLN WYQQKPGQSPKRLISLVSKL DSGIPDRFSGSGSGTDFTLTI SRLEPEDFAVYYCWQGSHFP YTFGQGTKLEIK | 15 |
| Y01h01v07 | EVTLKESGPTLVKPTQTLTLTCTV SGDSITSGYWNWIRQPPGKALEY LAYIRYSGRTYYNPSLKSRLTITR DTSKNQVVLTMTNMDPVDTATY YCASVYFTYWGQGTLVTVSS | 10 | DVVMTQSPLSLPVTLGQPAS ISCKSSQSLLDSDGKTYLNW FQQRPGQSPKRLISLVSKLD SGVPDRFSGSGSGTDFTLKIS RVEAEDVGVYYCWQGSHFP YTFGQGTKLEIK | 13 |
| Y01h01v08 | EVTLKESGPTLVKPTQTLTLTCTV SGDSITSGYWNWIRQPPGKALEY LAYIRYSGRTYYNPSLKSRLTITR DTSKNQVVLTMTNMDPVDTATY YCASVYFTYWGQGTLVTVSS | 10 | DVVMTQSPDSLAVSLGERA TINCKSSQSLLDSDGKTYLN WYQQKPGQSPKRLISLVSKL DSGVPDRFSGSGSGTDFTLTI SSLQAEDVAVYYCWQGSHF PYTFGQGTKLEIK | 14 |
| Y01h01v09 | EVTLKESGPTLVKPTQTLTLTCTV SGDSITSGYWNWIRQPPGKALEY LAYIRYSGRTYYNPSLKSRLTITR DTSKNQVVLTMTNMDPVDTATY YCASVYFTYWGQGTLVTVSS | 10 | EVVLTQSPGTLSLSPGERAT LSCKSSQSLLDSDGKTYLN WYQQKPGQSPKRLISLVSKL DSGIPDRFSGSGSGTDFTLTI SRLEPEDFAVYYCWQGSHFP YTFGQGTKLEIK | 15 |
| Y01h01v10 | EVQLVESGGGLVQPGRSLRLSCT VSGDSITSGYWNWFRQAPGKGL EYVGYIRYSGRTYYNPSLKSRFTI SRDTSKNIAYLQMNSLKTEDTAV YYCASVYFTYWGQGTLVTVSS | 11 | DVVMTQSPLSLPVTLGQPAS ISCKSSQSLLDSDGKTYLNW FQQRPGQSPKRLISLVSKLD SGVPDRFSGSGSGTDFTLKIS RVEAEDVGVYYCWQGSHFP YTFGQGTKLEIK | 13 |
| Y01h01v11 | EVQLVESGGGLVQPGRSLRLSCT VSGDSITSGYWNWFRQAPGKGL EYVGYIRYSGRTYYNPSLKSRFTI SRDTSKNIAYLQMNSLKTEDTAV YYCASVYFTYWGQGTLVTVSS | 11 | DVVMTQSPDSLAVSLGERA TINCKSSQSLLDSDGKTYLN WYQQKPGQSPKRLISLVSKL DSGVPDRFSGSGSGTDFTLTI SSLQAEDVAVYYCWQGSHF PYTFGQGTKLEIK | 14 |
| Y01h01v12 | EVQLVESGGGLVQPGRSLRLSCT VSGDSITSGYWNWFRQAPGKGL EYVGYIRYSGRTYYNPSLKSRFTI SRDTSKNIAYLQMNSLKTEDTAV YYCASVYFTYWGQGTLVTVSS | 11 | EVVLTQSPGTLSLSPGERAT LSCKSSQSLLDSDGKTYLN WYQQKPGQSPKRLISLVSKL DSGIPDRFSGSGSGTDFTLTI SRLEPEDFAVYYCWQGSHFP YTFGQGTKLEIK | 15 |

Nucleotide sequences of the anti-tau antibodies according to the examples of the present invention are summarized in Table 3 below.

**[Table 3]**

| **ADEL name** | **VH nucleotide sequence (SEQ ID NO)** | **VL nucleotide sequence (SEQ ID NO)** |
|---|---|---|
| Y01m01 | 16 | 21 |
| Y01h01v01 | 17 | 22 |
| Y01h01v02 | 17 | 23 |
| Y01h01v03 | 17 | 24 |
| Y01h01v04 | 18 | 22 |
| Y01h01v05 | 18 | 23 |
| Y01h01v06 | 18 | 24 |
| Y01h01v07 | 19 | 22 |
| Y01h01v08 | 19 | 23 |
| Y01h01v09 | 19 | 24 |
| Y01h01v10 | 20 | 22 |
| Y01h01v11 | 20 | 23 |
| Y01h01v12 | 20 | 24 |

The term "ADEL-Y01m" as used herein refers to a mouse antibody that specifically binds to a protein fragment which has, in the amino acid sequence of the tau protein represented by SEQ ID NO: 25, the 275^{th} to 286^{th} amino acid sequence section, with the 280^{th} amino acid acetylated. In addition, the term "ADEL-Y01h" as used herein refers to an antibody obtained by humanizing the ADEL-Y01m antibody.

The antigen-binding fragment of the antibody may be any one selected from the group consisting of Fab, scFv, F(ab')₂, and Fv. The fragment refers to antigen-binding domains except for a crystallizable region (Fc region) which has a function (effector function) to transmit stimulus caused by binding with an antigen to a cell, a complement, or the like, and the fragment may include a 3^{rd} generation fragment such as a single domain antibody and a minibody.

The fragment has advantages that it has a good penetration rate into the blood-brain barrier because the size of the fragment is smaller than that of IgG of a complete structure and that it has a low production cost because it can be produced in bacteria. In addition, since the antibody fragment lacks Fc, the antibody fragment is used in a case where a function to transmit stimulus caused by binding with an antigen to a cell, a complement, or the like is not desired. The fragment is suitable for diagnosis in a living body due to its short half-life in the human body. However, in a case where among amino acids constituting an antibody, some basic, acidic, or neutral amino acids are replaced with each other, an isoelectric point (pI) intrinsic to the antibody itself may change. Such change in isoelectric point of the antibody may lead to changes such as decreased toxic side effects *in vivo* of the antibody or increased water solubility. Therefore, in a case of a therapeutic antibody, in consideration of its affinity or structural form, IgG of a complete structure may be used.

The heavy chain variable region of the anti-tau antibody or antigen-binding fragment thereof may have a heavy chain variable region sequence containing any one amino acid sequence selected from SEQ ID NOs: 7 to 11, or it may have 95%, 97%, 98%, or 99% homology to the heavy chain variable region sequence.

The light chain variable region of the anti-tau antibody or antigen-binding fragment thereof may have a light chain variable region sequence containing any one amino acid sequence selected from SEQ ID NOs: 12 to 15, or it may have at least 95%, 97%, 98%, or 99% homology to the light chain variable region sequence.

The anti-tau antibody or antigen-binding fragment thereof may have at least 95%, 97%, 98%, or 99% homology to the heavy chain variable region sequence containing any one amino acid sequence selected from SEQ ID NOs: 7 to 11 and to the light chain variable region sequence containing any one amino acid sequence selected from SEQ ID NOs: 12 to 15.

The anti-tau antibody may be easily prepared by a known technique for preparing a monoclonal antibody. A monoclonal antibody may be prepared by obtaining a hybridoma using B lymphocytes from an immunized animal or by using a phage display technique. However, the method for preparing a monoclonal antibody is not limited thereto.

In an embodiment of the present invention, a monoclonal antibody of the anti-tau antibody may be easily prepared by preparing, as a hybridoma cell line, B lymphocytes that are obtained by injecting, into mice, a protein fragment (K280-ac) that has a 275^{th} to 286^{th} amino acid sequence section in the amino acid sequence of the wild-type tau protein of SEQ ID NO: 25 in which the 280^{th} amino acid is acetylated.

In another aspect of the present invention, there is provided a polynucleotide that encodes the anti-tau antibody or antigen-binding fragment thereof. In yet another aspect of the present invention, there is provided a polynucleotide that encodes a heavy or light chain of an antibody that includes the heavy chain variable region and the light chain variable region of the anti-tau antibody or antigen-binding fragment thereof. Specifically, the polynucleotide may include any one nucleotide sequence selected from the group consisting of SEQ ID NOs: 16 to 20 and/or any one nucleotide sequence selected from the group consisting of SEQ ID NOs: 21 to 24.

The polynucleotide may be any one selected from the following group:
a polynucleotide that includes the nucleotide sequence represented by SEQ ID NO: 16 and/or the nucleotide sequence represented by SEQ ID NO: 21;
a polynucleotide that includes the nucleotide sequence represented by SEQ ID NO: 17 and/or the nucleotide sequence represented by SEQ ID NO: 22;
a polynucleotide that includes the nucleotide sequence represented by SEQ ID NO: 17 and/or the nucleotide sequence represented by SEQ ID NO: 23;
a polynucleotide that includes the nucleotide sequence represented by SEQ ID NO: 17 and/or the nucleotide sequence represented by SEQ ID NO: 24;
a polynucleotide that includes the nucleotide sequence represented by SEQ ID NO: 18 and/or the nucleotide sequence represented by SEQ ID NO: 22;
a polynucleotide that includes the nucleotide sequence represented by SEQ ID NO: 18 and/or the nucleotide sequence represented by SEQ ID NO: 23;
a polynucleotide that includes the nucleotide sequence represented by SEQ ID NO: 18 and/or the nucleotide sequence represented by SEQ ID NO: 24;
a polynucleotide that includes the nucleotide sequence represented by SEQ ID NO: 19 and/or the nucleotide sequence represented by SEQ ID NO: 22;
a polynucleotide that includes the nucleotide sequence represented by SEQ ID NO: 19 and/or the nucleotide sequence represented by SEQ ID NO: 23;
a polynucleotide that includes the nucleotide sequence represented by SEQ ID NO: 19 and/or the nucleotide sequence represented by SEQ ID NO: 24;
a polynucleotide that includes the nucleotide sequence represented by SEQ ID NO: 20 and/or the nucleotide sequence represented by SEQ ID NO: 22;
a polynucleotide that includes the nucleotide sequence represented by SEQ ID NO: 20 and/or the nucleotide sequence represented by SEQ ID NO: 23; and
a polynucleotide that includes the nucleotide sequence represented by SEQ ID NO: 20 and/or the nucleotide sequence represented by SEQ ID NO: 24.

The polynucleotide may be modified by substitution, deletion, or insertion of at least one base, or a combination thereof. In a case where the nucleotide sequence is prepared by chemical synthesis, synthetic methods well known in the art may be used, such as those described in Engels and Uhlmann, Angew Chem IntEd Engl., 37:73-127, 1988. Such methods may include triester, phosphite, phosphoramidite and H-phosphate methods, PCR and other autoprimer methods, oligonucleotide syntheses on solid supports, and the like.

In still yet another aspect of the present invention, there is provided an expression vector comprising the polynucleotide. The expression vector may be a plasmid DNA, a phage DNA, or the like, and it may also include commercially developed plasmids (pUC18, pBAD, pIDTSAMRT-AMP, and the like), E. coli-derived plasmids (pYG601BR322, pBR325, pUC118, pUC119, and the like), Bacillus subtilis-derived plasmids (pUB110, pTP5, and the like), yeast-derived plasmids (YEp13, YEp24, YCp50, and the like), phage DNAs (Charon4A, Charon21A, EMBL3, EMBL4, λgt10, λgt11, λZAP, and the like), animal virus vectors (retrovirus, adenovirus, vaccinia virus, and the like), insect virus vectors (baculovirus), and the like. In the expression vector, expression level, modification, and the like of a protein vary depending on host cells, and thus it is preferred to choose and use a host cell most suitable for the purpose.

In still yet another aspect of the present invention, there is provided a host cell comprising the expression vector. The host cell for the transformed cell may include, but is not limited to, cells of mammalian, plant, insect, fungal, or bacterial origin. As the mammalian cells, CHO cells, F2N cells, CSO cells, BHK cells, Bowes melanoma cells, HeLa cells, 911 cells, AT1080 cells, A549 cells, HEK 293 cells, HEK293T cells, or the like may be used; however, the mammalian cells are not limited thereto. Any cell that is known to those skilled in the art and can be used as a mammalian host cell may be used.

In addition, in a case where the expression vector is introduced into a host cell, the following methods may be used: CaCl₂ precipitation, Hanahan's method in which a reducing substance called dimethyl sulfoxide (DMSO) is used in combination with CaCl₂ precipitation for increased efficiency, electroporation, calcium phosphate precipitation, protoplast fusion, agitation using silicon carbide fiber, Agrobacterium-mediated transformation, PEG-mediated transformation, dextran sulfate, lipofectamine, desiccation/inhibition-mediated transformation, and the like.

In still yet another aspect of the present invention, there is provided a method of producing an antibody or antigen-binding fragment thereof, the method comprising culturing the host cell. Specifically, the production method may comprise steps of i) culturing the host cell to obtain a culture and ii) collecting an antibody or antigen-binding fragment thereof from the culture.

The step of culturing the host cell may be performed using methods known in the art. Specifically, the culture may be carried out in a batch process or carried out continuously in a fed batch or repeated fed batch process.

The step of collecting the antibody or antigen-binding fragment thereof from the culture may be performed using methods known in the art. Specifically, the collection may be carried out with methods including centrifugation, filtration, extraction, spraying, drying, evaporation, precipitation, crystallization, electrophoresis, fractional dissolution (for example, ammonium sulfate precipitation), chromatography (for example, ion-exchange, affinity, hydrophobicity, or size-exclusion chromatography), and the like.

In still yet another aspect of the present invention, there is provided a hybridoma having accession number KCTC14155BP. The hybridoma having accession number KCTC14155BP may produce ADEL-Y01m. For the ADEL-Y01m, reference is made to the above description for the anti-tau antibody or antigen-binding fragment thereof. In still yet another aspect of the present invention, there is provided a pharmaceutical composition for preventing or treating a degenerative neurological disease, the pharmaceutical composition comprising, as an active ingredient, the anti-tau antibody or antigen-binding fragment thereof.

The degenerative neurological disease may be a tau protein-mediated neurological disease. Specifically, the tau protein-mediated neurological disease may be tauopathy, primary age-related tauopathy, chronic traumatic encephalopathy, progressive supranuclear palsy, corticobasal degeneration, frontotemporal dementia, Lytico-Bodig disease, Parkinsonism, subacute sclerosing meningitis, lead encephalopathy, tuberous sclerosis, ganglioglioma, gangliocytoma, meningioangiomatosis, subacute sclerosing panencephalitis, Hallervorden-Spatz disease, or lipofuscinosis.

In addition, the tauopathy may be Alzheimer's disease (AD), progressive supranuclear palsy (PSP), corticobasal degeneration (CBD), Pick's disease (PiD), a group of related disorders collectively termed frontotemporal dementia with Parkinsonism linked to chromosome 17 (FTDP-17), amyotrophic lateral sclerosis (ALS), Creutzfeld-Jakob disease (CJD), dementia pugilistica (DP), Gerstmann-Sträussler-Scheinker disease (GSSD), Lewy body disease, chronic traumatic encephalopathy (CTE), or Huntington's disease.

The pharmaceutical composition may further comprise one or more selected from the group consisting of pharmaceutically acceptable carriers, diluents, and adjuvants. Suitable carriers for the pharmaceutical composition are known to those skilled in the art and may include, but are not limited to, proteins, sugars, and the like. The carriers may be aqueous or non-aqueous solutions, suspensions, and emulsions. Examples of carriers which are non-aqueous solutions may include propylene glycol, polyethylene glycol, edible oil such as olive oil, and injectable organic ester such as ethyl oleate.

Examples of carriers which are aqueous solutions may include water, an alcohol/aqueous solution, an emulsion, or a suspension which includes saline and buffer media. Examples of carriers for parental administration may include a sodium chloride solution, Ringer's dextrose, dextrose and sodium chloride, lactated Ringer, or fixed oil. Examples of carriers for intravenous injection may include an electrolyte supplement such as one based on Ringer's dextrose, a liquid, and a nutritional supplement. Preservatives and other additives such as antimicrobial agents, antioxidants, chelating agents, and inert gases may be additionally present. Preferred preservatives may include formalin, thimerosal, neomycin, polymyxin B, and amphotericin B.

The pharmaceutical composition may further comprise an adjuvant (an immunomodulatory agent or an immunopotentiating agent). The adjuvant refers to a compound or mixture which enhances an immune response and/or accelerates an absorption rate after inoculation, and it may include any absorption accelerator. Acceptable adjuvants may include, but are not limited to, Freund's complete adjuvant, Freund's incomplete adjuvant, saponin, a mineral gel such as aluminum hydroxide, a surfactant such as lysolecithin, pluronic polyol, polyanions, a peptide, oil or a hydrocarbon emulsion, keyhole limpet hemocyanin, dinitrophenol, and the like.

The pharmaceutical composition may be administered through any one route of administration selected from the group consisting of intracerebral, intracerebroventricular, intraperitoneal, percutaneous, intramuscular, intradural, intravenous, subcutaneous, and nasal routes of administration, and it is preferably administered by an intracerebral, intracerebroventricular, intradural, or intraperitoneal route.

Specifically, the intracerebral, intracerebroventricular, or intradural administration may be useful in that there is no need to consider the problem of brain blood barrier (BBB) penetration. In addition, the intracerebral administration may be used in a case where it is desired to know an effect of a drug on a certain area in the brain by direct administration of a small amount of the drug to the area. The intracerebroventricular administration is a method of administering a drug to the cerebral ventricle, and it may be used in a case where it is desired to know an effect of the drug on the entire region of the brain. Furthermore, the intradural administration is a method of injecting a drug into the spinal canal by inserting a needle into the space around the spinal cord between two vertebrae in the lower part of the spine, and it may be used in a case where a drug is needed to provide a rapid or local effect on the brain, the spinal cord, or tissue layers (meninges) covering the brain or spinal cord.

According to an embodiment of the present invention, the antibody according to the present invention has effectively improved motor function cognitive function in a dementia-induced animal model through intraperitoneal administration as well as intracerebroventricular administration.

The pharmaceutical composition is administered in a pharmaceutically effective amount. The term "pharmaceutically effective amount" as used herein means an amount which is sufficient to enable the pharmaceutical composition to exhibit a therapeutic effect without causing side effects or serious or excessive immune responses. A level of the effective amount may vary depending on various factors including disorder to be treated, severity of the disorder, activity of a specific compound, route of administration, rate at which the protein is removed, duration of treatment, drugs used in combination or simultaneously with the protein, the individual's age, weight, sex, dietary habit, general health condition, and factors known in the medical field.

A dose of the pharmaceutical composition is desirably determined in consideration of the patient's age, sex, condition, degree of absorption of its active ingredient in the body, inactivation rate, and drugs used in combination. The pharmaceutical composition may be administered in an amount of 0.0001 mg/kg (body weight) to 300 mg/kg (body weight) based on the antibody or antigen-binding fragment thereof.

In yet another aspect of the present invention, there is provided a method for preventing or treating a degenerative neurological disease, the method comprising a step of administering the anti-tau antibody or antigen-binding fragment thereof to an individual who is expected to develop the degenerative neurological disease or has developed the degenerative neurological disease.

The anti-tau antibody or antigen-binding fragment thereof is as described above.

The dose of the anti-tau antibody or antigen-binding fragment thereof varies in its range depending on the patient's weight, age, sex, health condition, diet, time of administration, method of administration, rate of excretion and severity of disease. A single dose may be administered in an amount of about 0.001 mg/kg to 300 mg/kg on a daily or weekly basis. The effective amount may be decided at the discretion of a physician who treats the patient.

The effective amount of the anti-tau antibody or antigen-binding fragment thereof may vary depending on various factors such as the patient's age, weight, characteristics and degrees of symptoms, types of current treatments, the number of treatments, dosage form, and route of administration, and may be easily determined by experts in the relevant field.

The anti-tau antibody or antigen-binding fragment thereof may be administered together or sequentially with the above-mentioned pharmacological or physiological component, and it may also be administered in combination with an additional conventional therapeutic agent, in which the anti-tau antibody or antigen-binding fragment thereof may be administered sequentially or simultaneously with the conventional therapeutic agent. Such administration may be single or multiple administration. Taking all of the above factors into consideration, it is important to administer an amount which is a minimum amount and allows for maximum effects without side effects, and the amount may be easily determined by those skilled in the art.

In still yet another aspect of the invention, there is provided a composition for diagnosing a degenerative neurological disease, the composition comprising the anti-tau antibody or antigen-binding fragment thereof. The anti-tau antibody may be an antibody that binds to a modified tau protein. The antibody is described above.

In particular, it has been identified that acetylation of the 280^{th} amino acid in the amino acid sequence of the tau protein causes tau aggregation and a tau protein-mediated neurological disease. The anti-tau antibody or antigen-binding fragment thereof binds to an epitope containing 278^{th} to 284^{th} amino acids, with the 280^{th} amino acid acetylated, in the amino acid sequence of the tau protein represented by SEQ ID NO: 25, and thus the anti-tau antibody or antigen-binding fragment may be used to more effectively diagnose a tau protein-mediated neurological disease.

In a case where the antibody is used, assays for identifying an amount of the modified tau protein that binds thereto include, but are not limited to, western blotting, enzyme linked immunosorbent assay (ELISA), radioimmunoassay (RIA), radioimmunodiffusion, Ouchterlony immunodiffusion, rocket immunoelectrophoresis, immunohistological staining, immunoprecipitation assay, complement fixation assay, fluorescence activated cell sorter (FACS), protein chip method, and the like.

In still yet another aspect of the present invention, there is provided a kit for preventing, treating, or diagnosing a degenerative neurological disease, the kit comprising the anti-tau antibody or antigen-binding fragment thereof, the polynucleotide, the expression vector, or the host cell.

In still yet another aspect of the present invention, there is provided a diagnostic kit for a degenerative neurological disease, the kit comprising the anti-tau antibody or antigen-binding fragment thereof.

The kit can be used to measure an expression level of a modified tau protein from a sample of an individual, so as to diagnose a degenerative neurological disease. In addition, an antibody, as well as a composition, a solution, or a device which includes one or more other constituents and is suitable for an assay method may be included therein to measure an expression level of the modified tau protein.

A sample may be isolated from an individual by using a known process. The term "sample" as used herein includes, but is not limited to, a sample such as a tissue, a cell, blood, and plasma, each of which has a different expression level of the modified tau protein, and the like.

In addition, the step of measuring the protein may be carried out by western blotting, ELISA, radioimmunoassay, radioimmunodiffusion, Ouchterlony immunodiffusion, rocket immunoelectrophoresis, immunohistological staining, immunoprecipitation assay, complement fixation assay, FACS, or protein chip method. However, the present invention is not limited thereto.

Through the above assay methods, it is possible to compare an amount of an antigen-antibody complex formed in the normal control with an amount of an antigen-antibody complex formed in the individual, and to determine a tau protein-mediated neurological disease.

The term "antigen-antibody complex" as used herein means a conjugate of a modified tau protein with an antibody specific thereto, and an amount of the antigen-antibody complex formed can be quantitatively measured through a signal size of a detection label. Such a detection label may be selected from the group consisting of enzymes, fluorescent substances, ligands, luminescent substances, microparticles, redox molecules, and radioisotopes, but is not necessarily limited thereto. In a case where an enzyme is used as the detection label, available enzymes include β-glucuronidase, β-D-glucosidase, β-D-galactosidase, urease, peroxidase, alkaline phosphatase, and the like. The ligands include, but are not limited to, biotin derivatives, and the like.

In still yet another aspect of the present invention, there is provided a use of the anti-tau antibody or antigen-binding fragment thereof for preventing or treating a degenerative neurological disease.

In still yet another aspect of the present invention, there is provided a use of the anti-tau antibody or antigen-binding fragment thereof for preparing a medicament for prevention or treatment of a degenerative neurological disease.

Hereinafter, the present invention will be described in detail by way of examples. However, the following examples are intended to only illustrate the present invention, and the present invention is not limited thereto.

### I. Preparation of modified tau protein fragments and antibodies thereagainst

### Preparation Example 1. Preparation of modified tau protein fragments

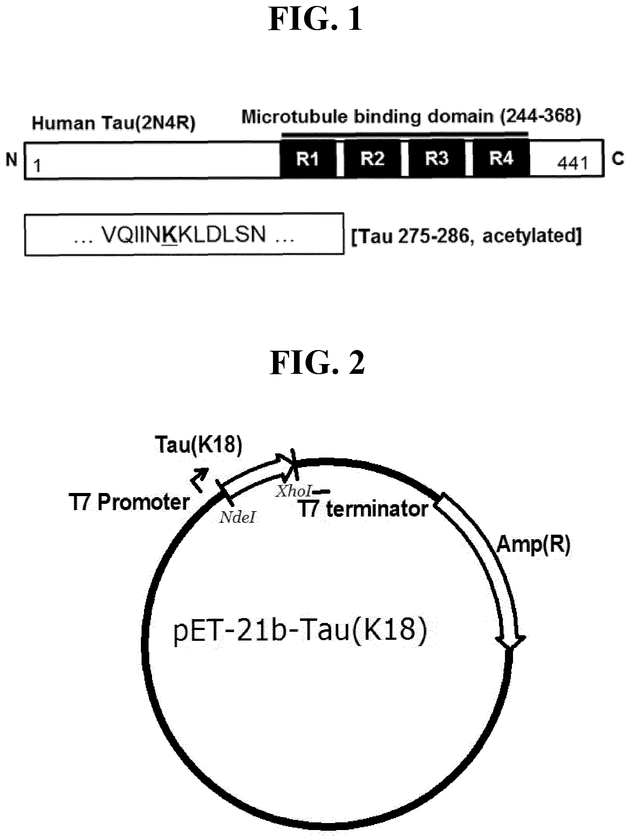

In order to prepare modified tau protein fragments, in the amino acid sequence of the tau protein (2N4R) which consists of 441 amino acids, tau protein fragments, which act in pathogenesis of Alzheimer's disease and show a cognitive function-improving effect upon active immunization of a tau gene-modified mouse model, were selected. As illustrated in Fig. 1, in the amino acid sequences of the tau protein fragments located in the microtubule-binding domain, a section consisting of 12 amino acids was designated.

Specifically, the protein fragment which has, in the wild type tau protein amino acid sequence of SEQ ID NO: 25, the 275^{th} to 286^{th} amino acid sequence section, with the 280^{th} amino acid acetylated, was designated as K280-ac. The K280-ac protein fragment was produced by requesting Peptron Inc. to produce the same.

In addition, a K280-ac protein fragment with keyhole limpet hemocyanin (KLH) bound at the N-terminus was produced by requesting ANYGEN Inc. (Nam-myeon, South Korea) to produce the same. The K280-ac protein fragment with KLH bound at the N-terminus was purified using the Shimadzu HPLC 10AVP system (purity of 91.8%) under a concentration gradient condition with 5% to 65% acetonitrile in 0.05% TFA.

Furthermore, the protein fragment of repeat domains (RDs) 1, 2, 3, and 4 which contains, in the wild type tau protein amino acid sequence of SEQ ID NO: 25, the 244^{th} to 372^{nd} amino acids (SEQ ID NO: 26) was designated as K18. A vector that contains a gene encoding the K18 protein fragment was transduced into *E. coli* cells to produce the K18 protein fragment.

Specifically, the K18 protein fragment was produced as follows. pET-21b-Tau (K18) expression vector (Fig. 2) was first transduced into *E. coli* BL21 (DE3), and cultured at a temperature of 37°C in LB medium containing ampicillin at 50 µg/ml concentration until the optical density (O.D.) at 600 nm wavelength reached 0.6 to 0.8. Thereafter, treatment with IPTG at 0.5 mM concentration was performed and the resultant was cultured at a temperature of 37°C for 3 hours and cultured at a temperature of 18°C for 18 hours.

After 18 hours, the formed single colony was cultured with starter culture in 5 ml of LB medium containing ampicillin at a concentration of 50 µg/ml under a condition at a temperature of 37°C and 220 rpm for about 16 hours. The starter culture was inoculated, at a ratio of 1:500, into an Erlenmeyer flask in which 5 L of LB medium containing ampicillin at a concentration of 50 µg/ml was placed and then cultured at a temperature of 37°C for 4 hours. Thereafter, the culture was centrifuged at 3,500 rpm for 30 minutes. Then, the supernatant was removed, and the remaining pellet was frozen at a temperature of -20°C.

The frozen pellet was resuspended in a lysis buffer (20 mM Tris-HCl, 1 mM phenylmethylsulfonyl fluoride, pH 8.0) at a rate of 25 ml/g. Cells were lysed for 30 minutes at a condition of 1,500 W using the JY99-IIDN ultrasonic homogenizer. Thereafter, the resultant was centrifuged under a condition at a temperature of 4°C and 15,000 rpm for 20 minutes, and the supernatant was filtered with a 0.45 µm filter.

The filtrate was filtered through a 5 mL His-Trap SP column equilibrated with Buffer A (20 mM Tris-HCl, pH 8.0). In order to remove non-specific binding, washing with 5 column volume (CV) of Buffer A was performed, and then elution was performed with a concentration gradient of 0 to 1 M NaCl using 10 CV of Buffer B (20 mM Tris-HCl, pH 8.0, 300 mM NaCl).

Fractions containing the K18 protein fragment were collected, and then the fractions were filtered through a 5 mL Hi-Trap SP column equilibrated with Buffer C (20 mM Tris-HCl, pH 8.0, 300 mM NaCl, 50 mM imidazole). In order to remove non-specific binding, washing with 10 CV of Buffer C was performed. Thereafter, elution was performed with 20 CV of Buffer D (20 mM Tris-HCl, pH 8.0, 300 mM NaCl, 100 mM imidazole). The eluate containing the purified K18 protein fragment was concentrated using a 10 kDa molecular weight centrifugal filter.

The acetylated K18 protein fragment (acK18) was prepared by reacting an acetylation buffer of (2-hydroxyethyl)-1-piperazine ethane sulfonic acid (HEPES, 10 mM, pH 7.4), 50 mM NaCl, 1.5 mM MgCl₂, 0.5 mM dithiothreitol (DTT), 2.5 mM EGTA, 0.1 mM EDTA, with the K18 protein fragment at 8 µM concentration, acetyl CoA at 125 µM concentration, and 0.5 µg of P300 enzyme which is acetyltransferase, at a temperature of 30°C for 3 hours.

An aggregate of the K18 protein fragments was prepared by dissolving the K18 protein fragments at 25 µM concentration in a 25 mM DTT solution for 1 hour at a temperature of 24°C, and performing agitation in a solution of (2-hydroxyethyl)-1-piperazine ethane sulfonic acid (HEPES, 10 mM, pH 7.4), NaCl (100 mM), heparin (25 mM) using the Eppendorf Thermomixer C under a condition at a temperature of 37°C and 700 rpm.

### Preparation Example 2. Preparation of antibody binding to modified tau protein fragment

An antibody that specifically binds to the K280-ac protein fragment prepared in Preparation Example 1 was produced by requesting AbFrontier, a South Korean mouse monoclonal antibody development company, to produce the same. B lymphocytes obtained by injecting the K280-ac protein fragment as an antigen into mice were produced as hybridoma cells. Thereafter, a cell line producing antibodies that specifically bind to the K280-ac protein fragment was screened through ELISA. In addition, phage display library screening was conducted by antibody development researchers at universities in South Korea, to develop antibodies that specifically bind to the K280-ac protein fragment.

As a result, one mouse hybridoma cell line (mouse hybridoma cell line) was finally selected, and candidates selected through the phage display library screening were excluded because they produce antibodies having a lower affinity than the mouse hybridoma cell line. The antibody produced from the mouse hybridoma cell line was checked *in vitro,* through western blotting and ELISA, for binding with the K280-ac protein fragment.

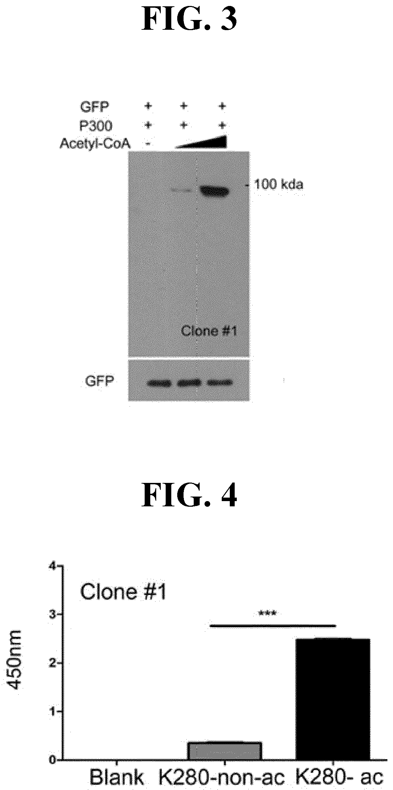

As a result, it was identified that the antibody produced from the #15E5 hybridoma cell line (clone #1) was an antibody that specifically bound to the K280-ac protein fragment (Figs. 3 and 4). The antibody produced from the #15E5 hybridoma cell line was finally selected and designated as ADEL-Y01m.

Starting from this mouse antibody, humanized antibodies were produced using conventionally known techniques including antibody variable region analysis, CDR investigation, molecular modeling, human germline antibody analysis, CDR grafting, and gene sequencing, and these humanized antibodies were designated ADEL-Y01h (v01 to v10).

On the other hand, the #15E5 hybridoma cell line was deposited at the Korean Collection for Type Cultures (KCTC) on March 18, 2020, and it was assigned microbial accession number KCTC14155BP.

### Experimental Example 1. Identification of antigen specific binding

In order to identify specific binding of the ADEL-Y01m antibody prepared in Preparation Example 2 to acetylated tau protein, the wild-type tau protein and the tau protein (Tau K280A) in which the 280^{th} amino acid lysine is replaced with alanine were acetylated *in vitro.* Here, acetylation of the wild-type Tau protein and the Tau K280A protein was performed in the same manner as in the preparation method of the acetylated K18 protein fragment in Preparation Example 1. Then, an antigen-antibody specific binding reaction of the ADEL-Y01m antibody was checked through western blotting.

First, the concentrations of the wild-type Tau protein and the Tau K280A protein were measured through Bradford Assay. Each of the proteins was mixed with 4X sample buffer (60 mM Tris-HCl [pH 6.8], 2% w/v SDS, 25% v/v glycerol, 14.4 mM v/v β-mercaptoethanol, and bromophenol blue). Thereafter, each protein was electrophoresed on an SDS-PAGE gel. Then, the electrophoresed gel was separated and transferred onto a PVDF membrane (BioRad, California, USA). Treatment with a blocking buffer (0.1% v/v Tween-20 [PBST], 3% w/v BSA, or 5% v/v skim milk in PBS buffer) was performed and reaction was allowed to proceed for 1 hour. Then, treatment with an anti-acetyl-lysine antibody (anti-acetyl-lysine, Cell Signaling, 9441) or ADEL-Y01m antibody as a primary antibody was performed and reaction was allowed to proceed at 4°C overnight. The next day, the PVDF membrane was washed three times with a washing buffer (0.1% v/v Tween-20 [PBST] in PBS buffer), and then treated with HRP-labeled anti-mouse IgG antibody (Bioxcell, BE0083). The PVDF membrane was treated with a chemiluminescent reagent (Thermo Fisher Scientific). Then, measurement and analysis were performed using x-ray film and ImageJ software (NIH, Bethesda, MD).

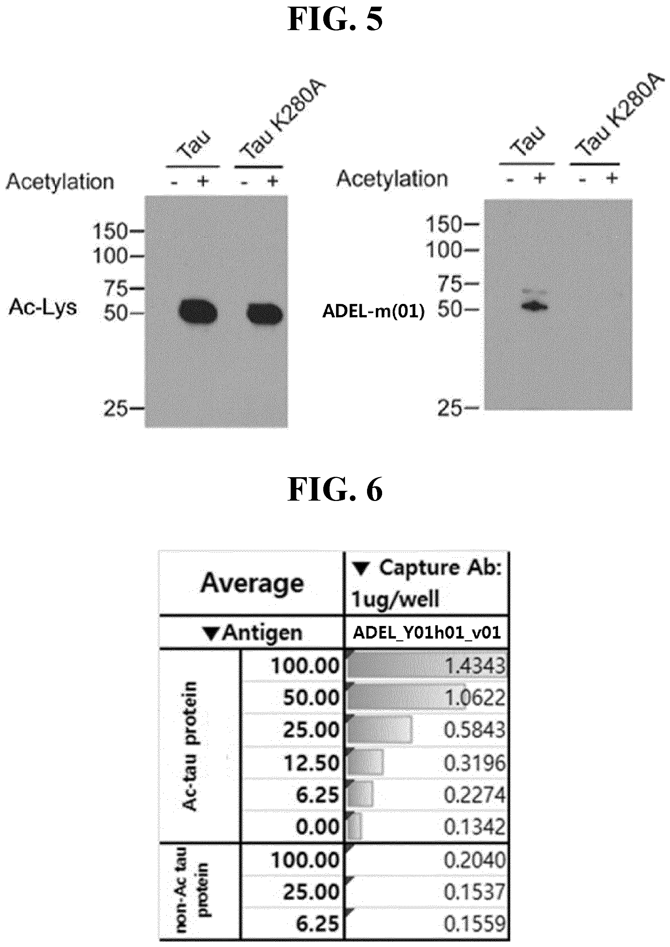

As a result, it was identified that the ADEL-Y01m antibody did not bind to the Tau K280A protein and to the acetylated Tau K280A protein (Fig. 5). From these results, it was identified that the ADEL-Y01m antibody was an antibody that specifically binds only to the tau protein in which the 280^{th} amino acid lysine was acetylated.

In addition, in order to identify specific binding with the ADEL-Y01h01_v01 antibody prepared in Preparation Example 2, western blotting was performed in the same manner as above using the K280-ac protein fragment and the K18 protein fragment. Here, the ADEL-Y01h01_v01 antibody and HRP-labeled anti-human IgG antibody (Bioxcell, BE0297) were used.

As a result, it was identified that the ADEL-Y01h01_v01 antibody exhibited a high antibody affinity for the K280-ac protein fragment (Fig. 6).

### Experimental Example 2. Identification of antigen-antibody affinity

An affinity between the K280-ac protein fragment prepared in Preparation Example 1 and the ADEL-Y01m antibody prepared in Preparation Example 2 was measured through the Octet^{®} K2 System (Fortebio).

The ADEL-Y01m antibody was fixed on AR2G biosensor according to the manufacturer's manual. The surface of the biosensor was hydrated with water for 10 minutes, and then activated using a mixture of 20 mM 1-ethyl-3-[3-dimethylaminopropyl] carbodiimide hydrochloride (EDC) and 10 mM N-hydroxysulfosuccinimide (s-NHS). The ADEL-YOlm antibody was fixed on the activated biosensor using 10 mM sodium acetate buffer (pH 5.0) and quenched with 1M ethanol amine (pH 8.0) for 5 minutes. The biosensor on which the ADEL-YOlm antibody is fixed was immersed in 1X PBS buffer (pH 7.4) for 60 seconds, to establish a baseline. Then, the biosensor was immersed for 60 seconds in wells containing different concentrations of the K280-ac protein fragment (antigen) or BSA-bound peptide. Thereafter, the biosensor was immersed again in 1X PBS buffer (pH 7.4) for 10 minutes so that dissociation occurs. The experimental data were evaluated by Octet Data Analysis (SW version: 9.0.0.10).

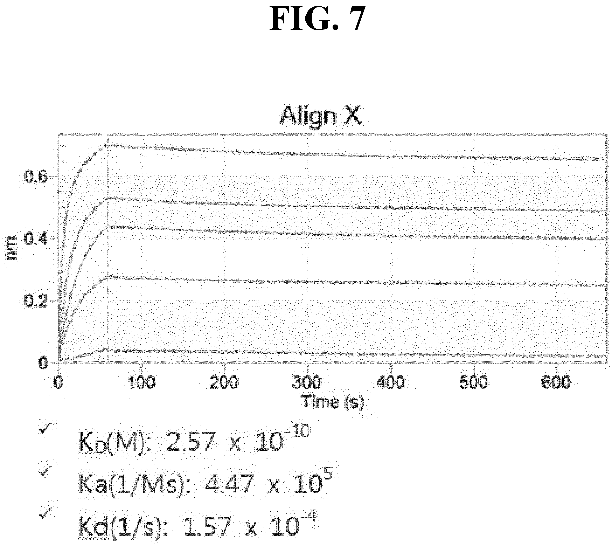

As a result, for the affinity between the ADEL-Y01m antibody and the K280-ac protein fragment, the antigen dissociation constant (Kd) value was measured as 2.57 × 10⁻¹⁰ M (Fig. 7).

In addition, binding of the K280-ac protein fragment prepared in Preparation Example 1 with the ADEL-Y01h (v01 to v12) antibodies prepared in Preparation Example 2 was measured by ELISA.

First, plates were treated with the K280-ac protein fragment at 250 ng/well and reaction was allowed to proceed at 4°C overnight. The next day, washing with a washing buffer was performed three times, and then treatment with a primary antibody was performed with 8 concentrations (2 µg, 1 µg, 0.5 µg, 0.25 µg, 0.12 µg, 0.06 µg, 0.03 µg, 0.01 µg). As a secondary antibody, HRP-labeled anti-human IgG antibody was used.

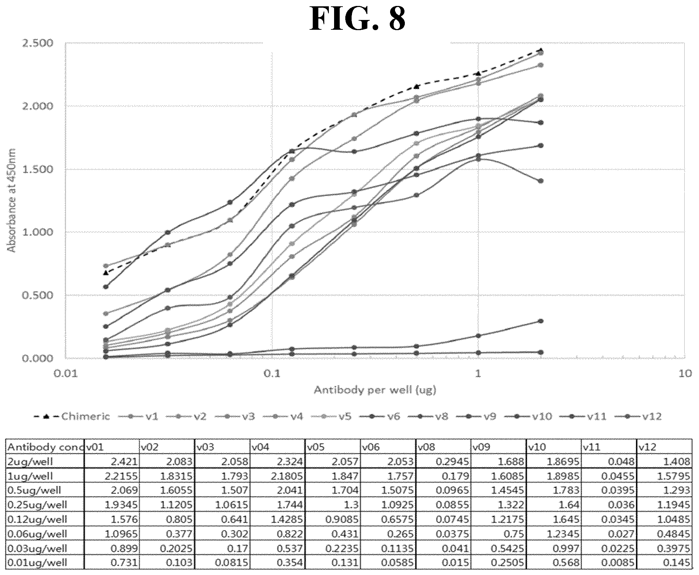

As a result, it was identified that the ADEL-Y01h (v01 to v10) antibodies exhibited a high antibody affinity for the K280-ac protein fragment (Fig. 8).

### II. Identification of therapeutic effects caused by ADEL-Y01m antibody using animal model

### Preparation Example 3. Preparation of dementia mouse model

JNPL3 mice expressing Tau-P301L mutant gene were purchased from Taconic, and back-crossed with C57bl/6 mice for five generations in the experimental animal room of the Asan Institute for Life Sciences, South Korea. Then, the mice were raised under a specific pathogen free (SPE) condition.

### Experimental Example 3. Identification of characteristics of dementia mouse model

The Tau-P301L dementia mouse model used in experiments exhibited a difference in accumulation of the tau protein in brain tissue with aging, as compared with the normal mice. Immunoblotting using the ADEL-Y01m antibody prepared in Preparation Example 2 was conducted.

The brains were extracted from normal mice and Tau-P301L dementia mice, and then soluble and insoluble proteins were separated through Sarkosyl fractions from brain tissues. Brain samples were homogenized with RABuffer B (100 mM MES, 0.75 M NaCl, 1 mM EGTA, 0.5 mM MgSO₄, 2 mM DTT, pH 6.8 + protease/phosphatase inhibitors). The samples were incubated on ice for 20 minutes and centrifuged at 9,000 rpm for 20 minutes at 4°C. After centrifugation, the supernatant was collected (soluble proteins). The pellet was homogenized with an extraction buffer (1% Sarkosyl, 10 mM Tris, 10% sucrose, 0.85 M NaCl, 1 mM EGTA, pH 7.4). The samples were incubated for 1 hour at room temperature and centrifuged at 13,000 rpm for 20 minutes at 4°C. After centrifugation, the supernatant was collected (tau aggregates). The separated proteins were subjected to western blotting in the same manner as in Experimental Example 1.

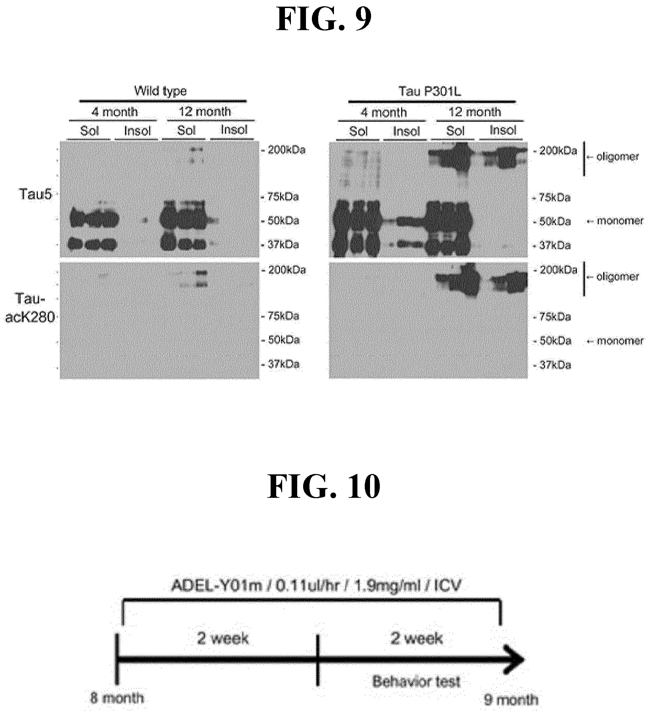

As a result, it was identified that a 12-month-old dementia mouse model exhibited an increase in the number of acetylated tau proteins in the brain tissues. In particular, it was identified that a 4-month-old dementia mouse model exhibited an expression level of the tau protein similar to that in the normal mice, whereas a 12-month-old dementia mouse model showing severe dementia symptoms exhibited an expression level of the tau protein which was about 2 times as compared with the normal mice (Fig. 9).

### Experimental Example 4. Identification of behavior-improving effect caused by ADEL-Y01m antibody: Intracerebroventricular administration

In order to identify therapeutic effects caused by the ADEL-Y01m antibody, the ADEL-Y01m antibody was administered to the lateral ventricle of the Tau-P301L dementia mice prepared in Preparation Example 3 using an osmotic pump according to the experimental schedule illustrated in Fig. 10.

### Experimental Example 4.1. Identification of behavior-improving effect through nesting building test

A nest building test which can identify a multimodal brain function of the mice was performed.

Specifically, two-thirds of the litter in a cage where the mice were raised was removed and four layers of sterile cotton (5 cm × 5 cm) were placed in the cage. One mouse was allowed to enter one cage. The next morning, a degree of completion of the nest was analyzed.

The score according to nest completion was calculated by dividing it into 1 to 5 points. For a case where the provided sterile cotton is maintained at 90% or higher, or hardly touched, the case was calculated as 1 point; and for a case where the sterile cotton is maintained at about 50% to 90% and partially torn, the case was calculated as 2 points. For a case where the sterile cotton is torn at 50% or higher and spread around the cage, the case was calculated as 3 points; and for a case where the sterile cotton is torn at 90% or higher and gathered on the side of the cage, the case was calculated as 4 points. For a case where the sterile cotton is completely torn and a complete nest shape is formed on the side of the cage, the case was calculated as 5 points.

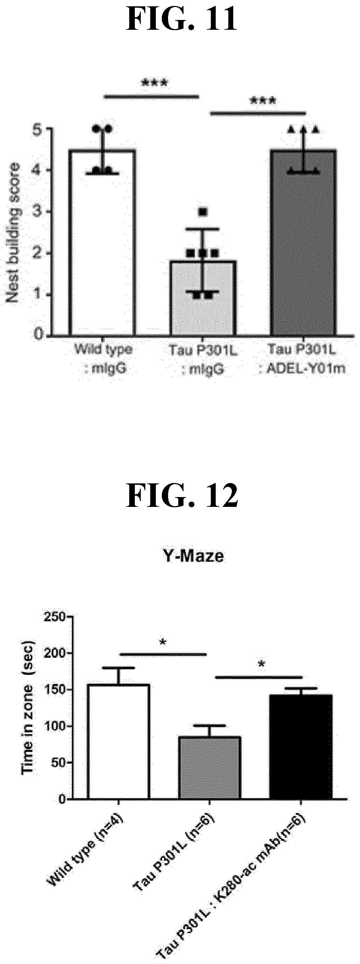

As a result, it was identified that the Tau-P301L dementia mice exhibited about 60% decrease in building score as compared with the normal mice and that the dementia mice (Tau-P301L-ADEL-Y01m) into which the ADEL-YOlm antibody had been administered exhibited a similar building score to the normal mice (Fig. 11).

### Experimental Example 4.2. Identification of behavior-improving effect through Y-maze test

A Y-maze test was performed to identify restoration of cognitive function. The Y-maze test is an experiment to identify short-term memory capacity. Specifically, A, B, and C zones were set in a Y-shaped box, and the mice were allowed to freely move for 5 minutes in a state where the B zone was blocked. One hour later, the state where the B zone was blocked was eliminated, and movement of the mice was observed for 5 minutes in a state where all zones were open.

As a result, in a case of the Tau-P301L mice, the time spent staying in the A or C zone was 100 seconds or shorter which was about 50% decreased as compared with the normal mice. On the other hand, in a case of the Tau-P301L-ADEL-Y01m dementia mice into which the ADEL-YOlm antibody had been administered, the time spent staying in the A or C zone was around 150 seconds which was similar to the normal mice. From these results, it was identified that the short-term memory capacity of the Tau-P301L-ADEL-Y01m dementia mice is restored (Fig. 12).

### Experimental Example 4.3. Identification of behavior-improving effect through water maze test

An underwater maze test was performed to evaluate a learning and memory ability of the mice. For the equipment and program used for the underwater maze test, behavioral equipment possessed by the experimental animal room of the Asan Institute for Life Sciences was used. Specifically, the underwater maze test took a total of 5 days. The mice were trained for 4 days in a state where a platform and a visual cue for the platform location were placed.

The learning was conducted by filling a swimming pool (1.4 m in diameter, 45 cm in depth) having a platform with water (29 ± 0.5°C) to a depth of about 26.5 cm, and then 1.5 L of whole milk powder was added to make water cloudy so that the mice could remember the platform with the cue only. Then, the mice were adapted to freely swim for 60 seconds and allowed to rest on the platform for 1 minute. The experiment was performed over a total of 12 times with 4 sets at three different starting positions. At the end of each test, 30 seconds of break time was given. At the end of one set, 30 to 45 minutes of break time was given.

After 4 days of learning, the test was performed on the 5^{th} day. In the experiment for evaluation, the platform was not placed, and the number of staying in the area where the platform had been placed was measured in order to identify whether the mice visited well the platform location using the cue only. In addition, the mice were allowed to arrive at the location where the platform had been placed within one minute. If the mice fail to arrive at the platform location within one minute, the mice were removed out of the swimming pool.

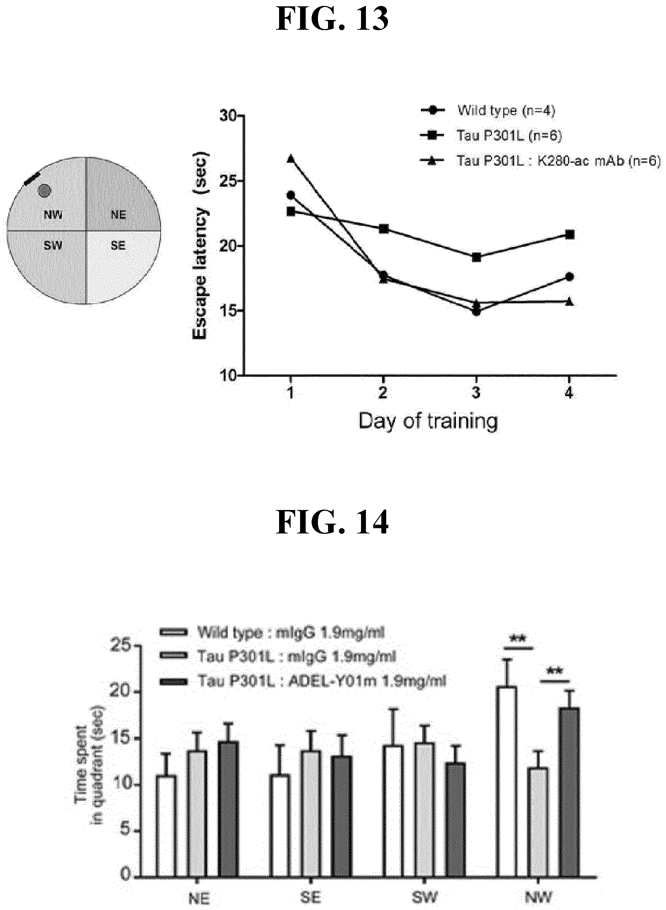

First, the time taken for the mice to find the platform during the 4 days of learning was measured. As a result, in a case of the Tau-P301L mice, the time taken to find the platform was 20 seconds or longer during the 4 days. On the other hand, in a case of the normal mice and the Tau-P301L-ADEL-Y01m dementia mice into which the ADEL-Y01m antibody had been administered, the time taken to find the platform was decreased to 20 seconds or shorter starting from the 2^{nd} day (Fig. 13).

In addition, on the 5^{th} day, evaluation was performed on the time spent staying in the NW zone where the platform had been placed. As a result, it was identified that the Tau-P301L mice stay in the NW zone for 10 seconds or shorter, whereas the normal mice and the Tau-P301L-ADEL-Y01m dementia mice into which the ADEL-Y01m antibody has been administered stay in the NW zone for 20 seconds or shorter (Fig. 14).

### Experimental Example 4.4. Identification of behavior-improving effect through grip strength test

A grip strength test was performed to identify muscle strength of the mice. For an iron bundle used in experiments, behavioral equipment possessed by the Asan Institute for Life Sciences was used. Here, an iron bundle that weighs 40 g was used for the iron bundle. The mice prepared in Preparation Example 3 were held by the tail end, and the forefeet of the mice were caused to be placed on the 40 g iron bundle so that the mice can grab the iron bundle. Then, the mice were lifted up to a height of about 30 cm in a state where the tail thereof was held. The time until the mice missed the iron bundle was measured and comparison was made for the respective mice.

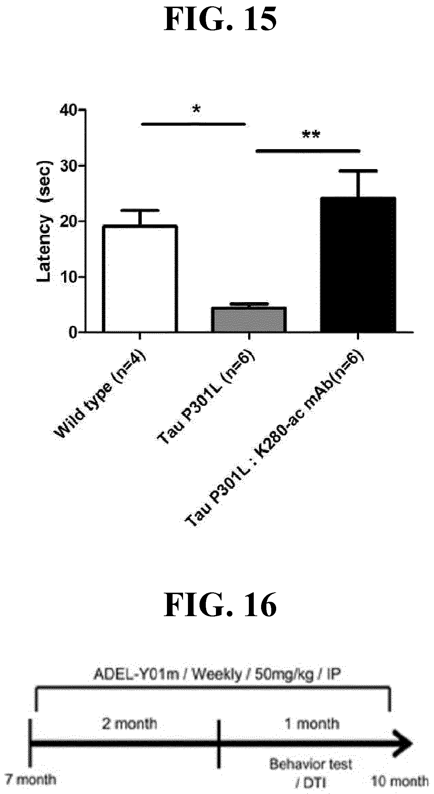

As a result, the normal mice exhibited a measured time for grab strength of 20 seconds or shorter, and the Tau-P301L dementia mice exhibited a measured time for grab strength of 5 seconds or shorter. On the contrary, in a case of the Tau-P301L-ADEL-Y01m dementia mice into which the ADEL-Y01m antibody had been administered, a measured time for grab strength was 20 seconds or longer which was longer than that in the normal mice (Fig. 15). From these results, it was identified that the motor ability of the Tau-P301L-ADEL-Y01m dementia mice into which the ADEL-Y01m antibody has been administered is restored.

### Experimental Example 5. Identification of behavior-improving effect of ADEL-Y01m antibody: Intraperitoneal administration

In order to identify therapeutic efficacy of the ADEL-Y01m antibody, the ADEL-Y01m antibody was administered intraperitoneally to the Tau-P301L dementia mice prepared in Preparation Example 3 for 3 months according to the experimental schedule illustrated in Fig. 16.

### Experimental Example 5.1. Identification of behavior-improving effect through nesting building test

A nest building test which can identify a multimodal brain function of the mice was performed in the same manner as Experimental Example 4.1.

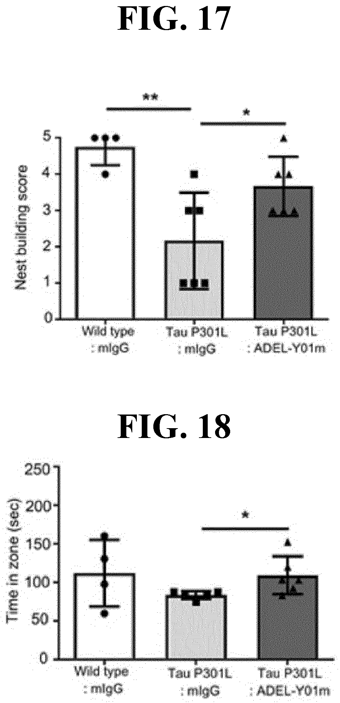

As a result, it was identified that the Tau-P301L dementia mice exhibit about 60% decrease in building score as compared with the normal mice and that the Tau-P301L-ADEL-Y01m dementia mice into which the ADEL-YOlm antibody has been administered exhibit a similar building score to the normal mice (Fig. 17).

### Experimental Example 5.2. Identification of behavior-improving effect through Y-maze test

A Y-maze test was performed, in the same manner as in Experimental Example 4.2, to identify restoration of cognitive function. As a result, in a case of the Tau-P301L mice, the time spent staying in the A or C zone was around 80 seconds which was about 50% decreased as compared with the normal mice. On the other hand, in a case of the Tau-P301L-ADEL-Y01m dementia mice into which the ADEL-Y01m antibody had been administered, the time spent staying in the A or C zone was around 110 seconds which was similar to the normal mice. From these results, it was identified that the short-term memory capacity of the Tau-P301L-ADEL-Y01m dementia mice is restored (Fig. 18).

### Experimental Example 5.3. Identification of behavior-improving effect through water maze test

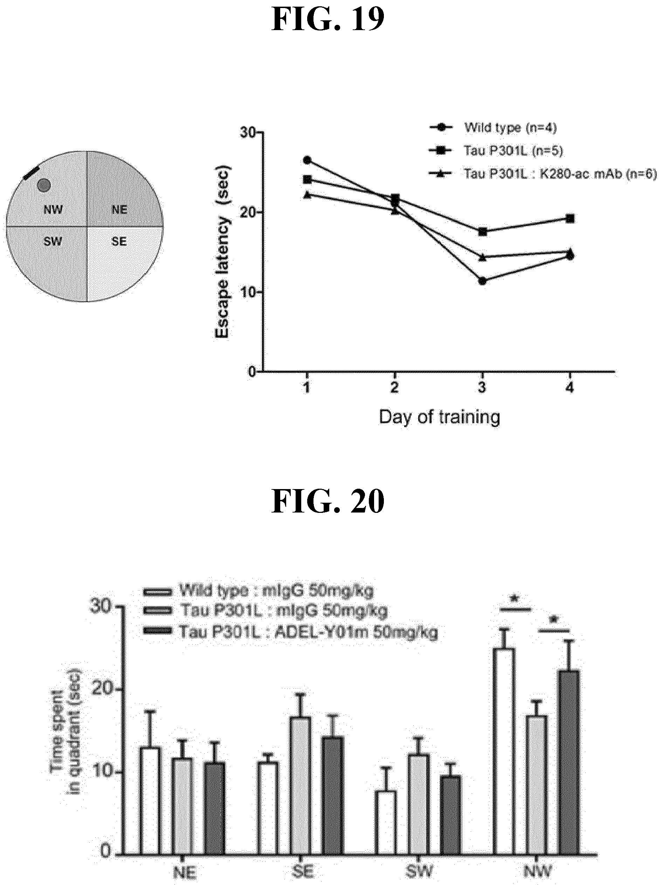

An underwater maze test was performed in the same manner as in Experiment 4.3. The time taken for the mice to find the platform during the 4 days of learning was measured. As a result, in a case of the Tau-P301L mice, the time taken to find the platform was 20 seconds or shorter during the 4 days. On the other hand, in a case of the normal mice and the Tau-P301L-ADEL-Y01m dementia mice into which the ADEL-Y01m antibody had been administered, the time taken to find the platform was decreased to 15 seconds or shorter starting from the 3^{rd} day (Fig. 19).

In addition, on the 5^{th} day, evaluation was performed on the time spent staying in the NW zone where the platform had been placed. As a result, it was identified that the Tau-P301L mice stay in the NW zone for 20 seconds or shorter, whereas the normal mice and the Tau-P301L-ADEL-Y01m dementia mice into which the ADEL-Y01m antibody has been administered stay in the NW zone for about 25 seconds (Fig. 20).

### Experimental Example 6. Identification I of specific binding between ADEL-Y01m antibody and modified tau protein expressed in mouse tissue

Cerebral cortices and hippocampal tissues of the normal mice and the Tau-P301L dementia mice prepared in Preparation Example 3 were subjected to immunoprecipitation (IP) and western blotting using IgG and ADEL-Y01m antibody.

First, a sample buffer was prepared by adding, to distilled water, 1% Tx-100, 100 mM NaCl, and 50 mM HEPES, and adjusting the pH to pH 7.4. Subsequently, the sample buffer was used to resuspend the cerebral cortices and hippocampal tissues of the normal mice and the Tau-P301L dementia mice, so that resuspended samples were obtained. Each of the obtained samples was treated with IgG or ADEL-Y01m antibody, and reaction was allowed to proceed for 24 hours. Then, the sample was treated with 100 µl of protein G-agarose beads (GE Healthcare Life Sciences), and reaction was allowed to proceed at 4°C overnight. The next day, centrifugation was performed at 6,000 rpm for 1 minute at 4°C, to collect the sample, and washing was performed three times with PBS containing 0.1% Triton X-100. The sample was treated with 1X sample buffer, and subjected to western blotting. Here, the western blotting was performed in the same manner as in Experimental Example 1, and IgG, ADEL-Y01m antibody, and HRP-labeled anti-mouse IgG antibody were used.

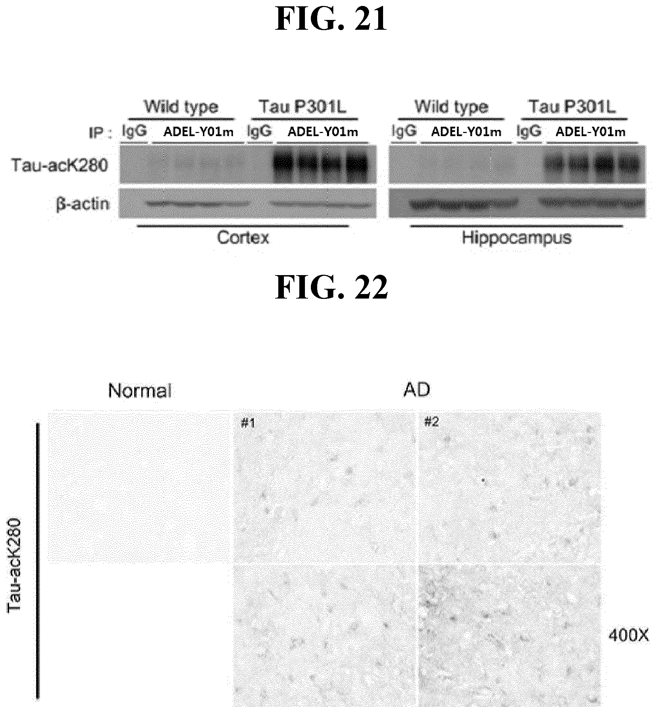

As a result, it was identified that there was specific binding reaction between the ADEL-Y01m antibody and the tau protein (Tau-acK280), in which the 280^{th} amino acid lysine was acetylated, in the cerebral cortices and hippocampal tissues of the Tau-P301L dementia mice. In addition, from these results, it was identified that the ADEL-Y01m antibody bound well to a modified tau protein expressed in the brain tissue *in vivo* (Fig. 21).

### Experimental Example 7. Identification II of binding between ADEL-Y01m antibody and modified tau protein expressed in human tissue

In order to identify specific binding between the ADEL-Y01m antibody and a modified tau protein expressed in human brain tissue, temporal cortices in brain tissues of normal elderly and patients with Alzheimer's disease were subjected to immunohistochemical staining using the ADEL-Y01m antibody.

First, each of the temporal cortices in brain tissues of normal elderly and patients with Alzheimer's disease was sectioned and attached to a slide glass. The brain tissues were provided by the Netherlands Brain Bank. The section was washed with 1X PBS, and permeation was performed with 1X PBS containing 0.1% Triton X-100 at room temperature for 10 minutes. Then, the section was washed and blocked with PBS containing 3% BSA at room temperature for 1 hour. The section was washed with 1X PBS and reacted with the ADEL-Y01m antibody at 4°C overnight.

The next day, for diaminobenzidine (DAB) staining, the section obtained by being reacted with the primary antibody overnight was washed with 1X PBS. The section was reacted with biotinylated anti-mouse IgG antibody (Vector Laboratories, California, USA) as a secondary antibody at room temperature for 1 hour. The section was washed with 1X PBS, treated with avidin-biotin-peroxidase complex (Vector Laboratories, California, USA), and allowed to react. The brain tissue section was allowed to react for 10 minutes in DAB solution, and then washed with 1X PBS. The section was mounted on Canada balsam (Sigma-Aldrich). The section was imaged using a Zeiss microscope (Carl Zeiss, Oberkochen, Germany) and the images were processed using the AxioVision Imaging System.

As a result, staining with the ADEL-Y01m antibody was not observed in the temporal cortices of normal elderly, whereas staining with the ADEL-Y01m antibody was observed in the temporal cortices of patients with Alzheimer's disease. In addition, from these results, it was identified that the ADEL-Y01m antibody bound well to a modified tau protein expressed in the brain tissue *in vivo* (Fig. 22).

In addition, the brain tissues of normal elderly and patients with Alzheimer's disease were analyzed through western blotting. Here, the western blotting was performed in the same manner as in Experimental Example 1, and anti-Tau5 antibody (Invitrogen, AHB0042), anti-acetyl-K280 antibody (Anaspec, AS-56077), anti-amyloid-β antibody (Covance, SIG-39300), anti-GAPDH antibody (Millipore, MAB374), and HRP-labeled anti-human IgG antibody were used.

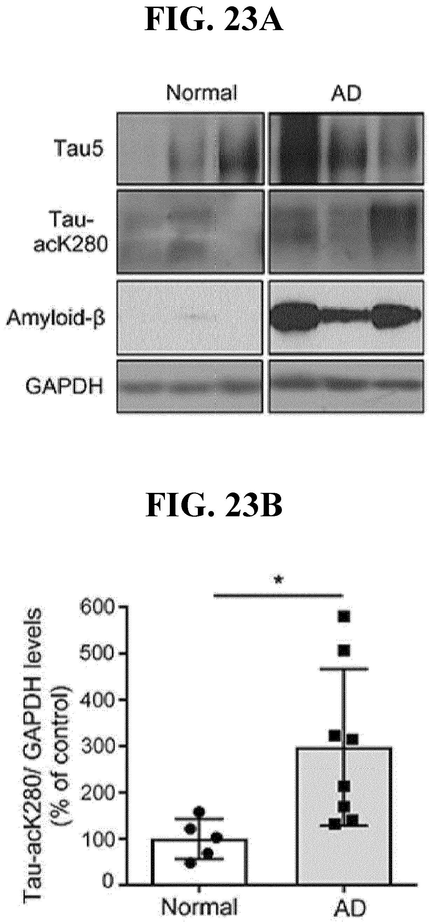

As a result, increased expression levels of the entire tau protein, acetylated tau protein, and amyloid-β were observed in the brain tissues of patients with Alzheimer's disease as compared with the brain tissues of normal elderly (Fig. 23A). In particular, about 3 times more acetylated tau protein (Tau-acK280) was observed in the brain tissues of patients with Alzheimer's disease (Fig. 23B).

### Experimental Example 8. Identification of tauopathy-alleviating effect following intracerebroventricular administration of ADEL-Y01m antibody

In order to identify a tauopathy-alleviating effect following administration of the ADEL-Y01m antibody, brain tissues of the Tau-P301L dementia mice into which IgG or ADEL-Y01m antibody had been intracerebroventricularly administered and the normal mice were extracted, and expression levels of the entire tau protein (Tau5), acetylated tau protein (Tau-acK280), and tau (Ser396) obtained by phosphorylating serine, the 396^{th} amino acid, in the amino acid sequence of the tau protein were checked through western blotting. Here, the western blotting was performed in the same manner as in Experimental Example 1, and anti-Tau5 antibody (Invitrogen, AHB0042), anti-pSer396 antibody (Thermo, 44-752G), anti-acetyl-K280 antibody (Anaspec, AS-56077), and HRP-labeled anti-mouse IgG antibody (Bioxcell, BE0083) were used.

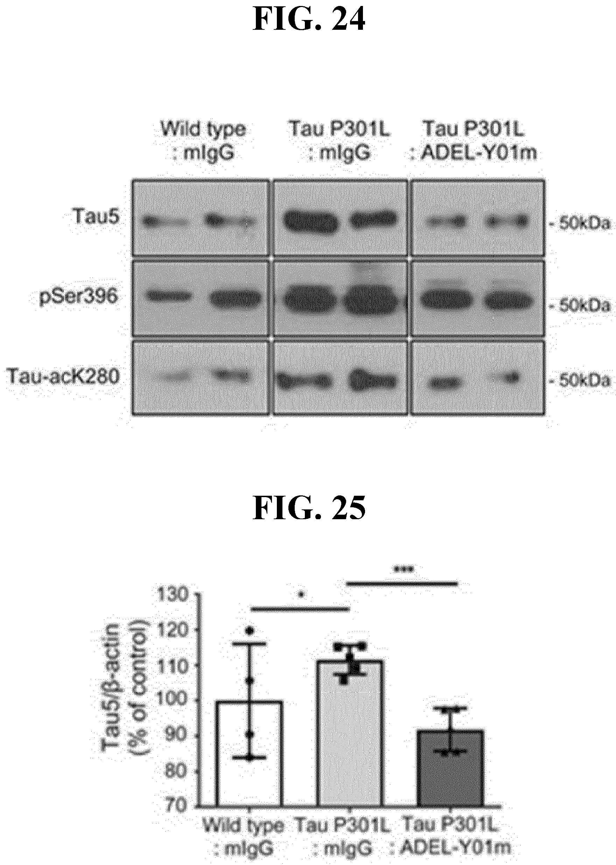

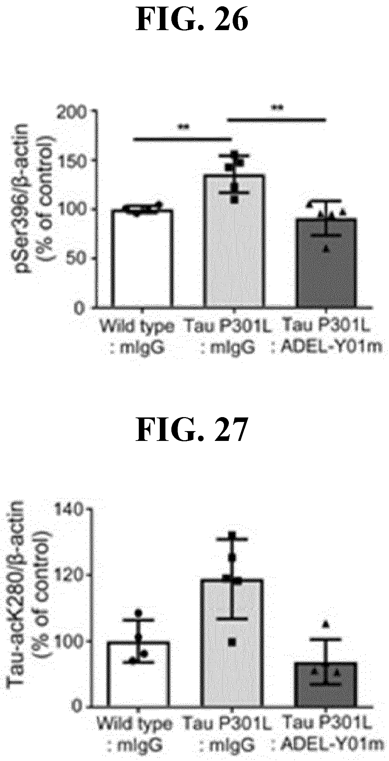

As a result, it was identified that the entire tau protein, the acetylated tau protein, and the phosphorylated tau protein are present in higher amounts in the brain tissues of Tau-P301L dementia mice, into which IgG has been intracerebroventricularly administered, than in the brain tissues of normal mice. On the other hand, the entire tau protein, the acetylated tau protein, and the phosphorylated tau protein were present in lower amounts in the brain tissues of Tau-P301L dementia mice, into which the ADEL-Y01m antibody had been intracerebroventricularly administered, than in the brain tissues of Tau-P301L dementia mice, into which IgG had been intracerebroventricularly administered (Figs. 24 to 27).

In addition, immunohistochemical staining was performed to identify expression levels of tau protein (pSer202/pThr205), obtained by phosphorylating the 202^{nd} amino acid serine and the 205^{th} amino acid threonine in the amino acid sequence of the tau protein, and phosphorylated tau (pT231) in the brain tissues of the mice. Here, the immunohistochemical staining was performed in the same manner as in Experimental Example 7, and an anti-AT8 antibody (Thermo, MN1020) and anti-pT231 antibody (Thermo, MN1040) were used as primary antibodies.

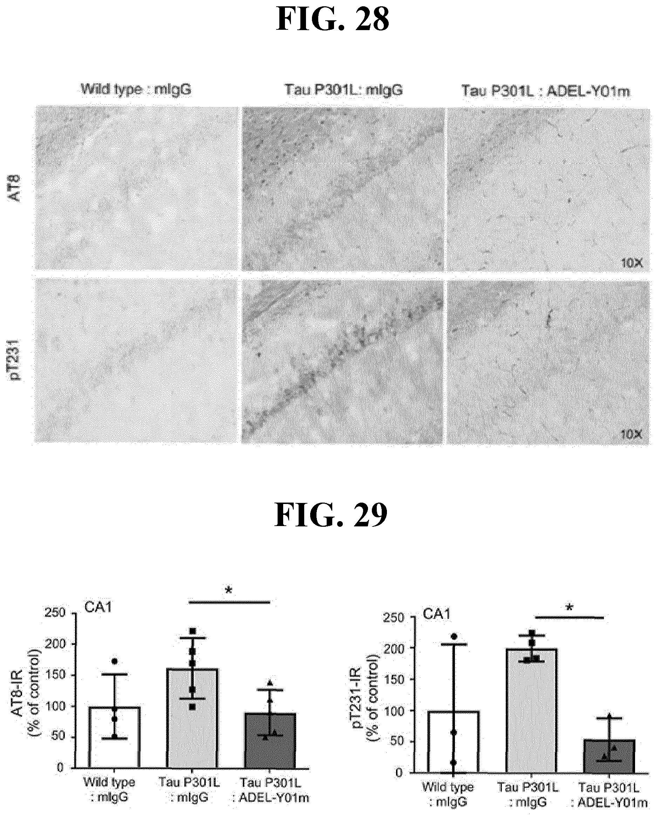

As a result, an increased expression level of the phosphorylated tau protein was observed in the brain tissues of Tau-P301L dementia mice, into which IgG had been intracerebroventricularly administered, as compared with the brain tissues of normal mice. On the other hand, a decreased expression level of the phosphorylated tau protein was observed in the brain tissues of Tau-P301L dementia mice, into which the ADEL-Y01m antibody had been intracerebroventricularly administered, as compared with the brain tissues of Tau-P301L dementia mice, into which IgG had been intracerebroventricularly administered (Figs. 28 and 29).

### Experimental Example 9. Identification of tauopathy-alleviating effect following intraperitoneal administration of ADEL-Y01m antibody

In order to identify a tauopathy-alleviating effect following administration of the ADEL-Y01m antibody, brain tissues of the Tau-P301L-ADEL-Y01m dementia mice, into which IgG or ADEL-Y01m antibody had been intraperitoneally administered in an amount of 50 mg/kg, and the normal mice were extracted, and expression levels of the entire tau protein (Tau5), acetylated tau, and phosphorylated tau (pT231) were checked through semi-denaturating western blotting. Here, the semi-denaturating western blotting was performed in the same manner as the western blotting in Experimental Example 1, except that the mouse brain tissue was mixed with a 2X laemmli sample buffer without β-mercaptoethanol. In addition, anti-Tau5 antibody (Invitrogen, AHB0042), anti-pT231 antibody (Thermo, MN1040), anti-acetyl-K280 antibody (Anaspec, AS-56077), and HRP-labeled anti-mouse IgG antibody (Bioxcell, BE0083) were used.

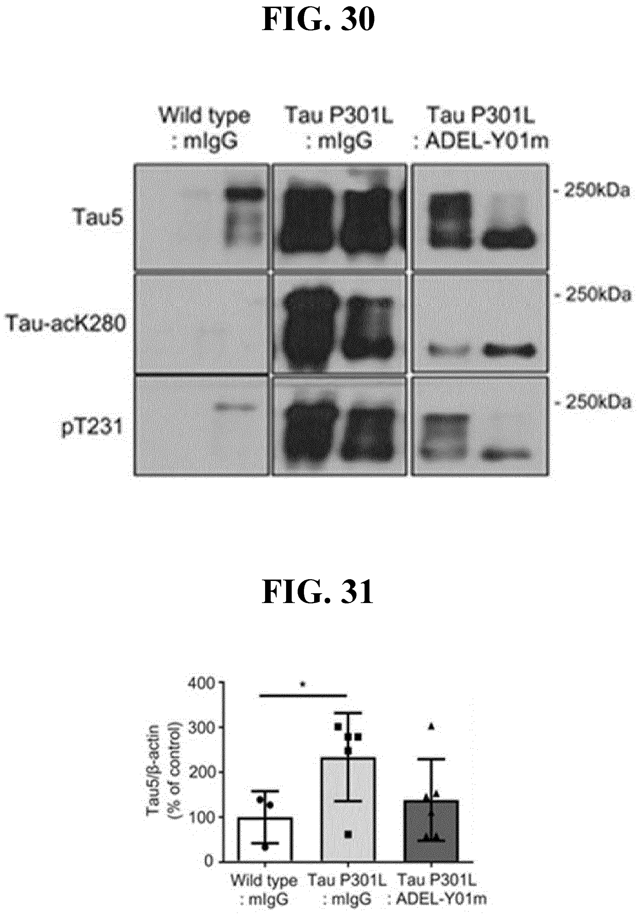

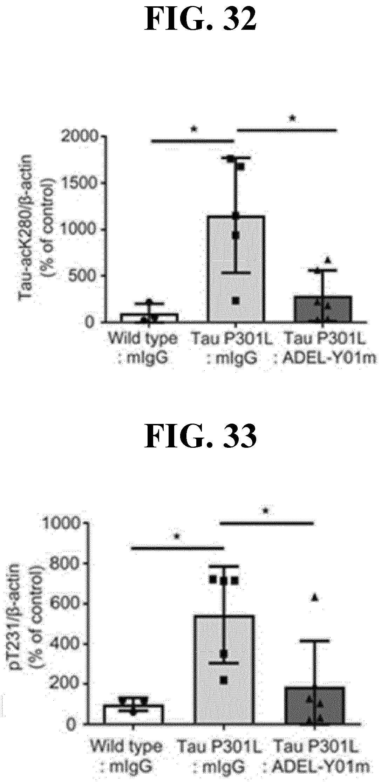

As a result, it was identified that an oligomeric form of the entire tau protein, phosphorylated tau protein, and acetylated tau protein were present in higher amounts in the brain tissues of the Tau-P301L dementia mice, into which IgG had been intraperitoneally administered, than in the brain tissues of the normal mice. On the other hand, the entire tau protein, the phosphorylated tau protein, and the acetylated tau protein were present in remarkably lower amounts in the brain tissues of the Tau-P301L-ADEL-YOlm dementia mice, into which the ADEL-YOlm antibody had been intraperitoneally administered, than in the brain tissues of the Tau-P301L dementia mice, into which IgG had been intraperitoneally administered (Figs. 30 to 33).

### Experimental Example 10. Identification of synapse-improving effect caused by ADEL-Y01m antibody: Intracerebroventricular administration

In order to identify a synapse-improving effect following administration of the ADEL-Y01m antibody, brain tissues of the Tau-P301L dementia mice, into which IgG or ADEL-Y01m antibody had been intraventricularly administered in an amount of 50 mg/kg, and the normal mice were extracted, and western blotting was performed on synapse-related proteins. Here, the western blotting was performed in the same manner as in Experimental Example 1, and anti-PSD95 antibody (Abcam, ab2723), anti-synapsin-1 antibody (Chemicon, MAB355), anti-β-actin antibody (Sigma, A5441), and HRP-labeled anti-mouse IgG antibody (Bioxcell, BE0083) were used.

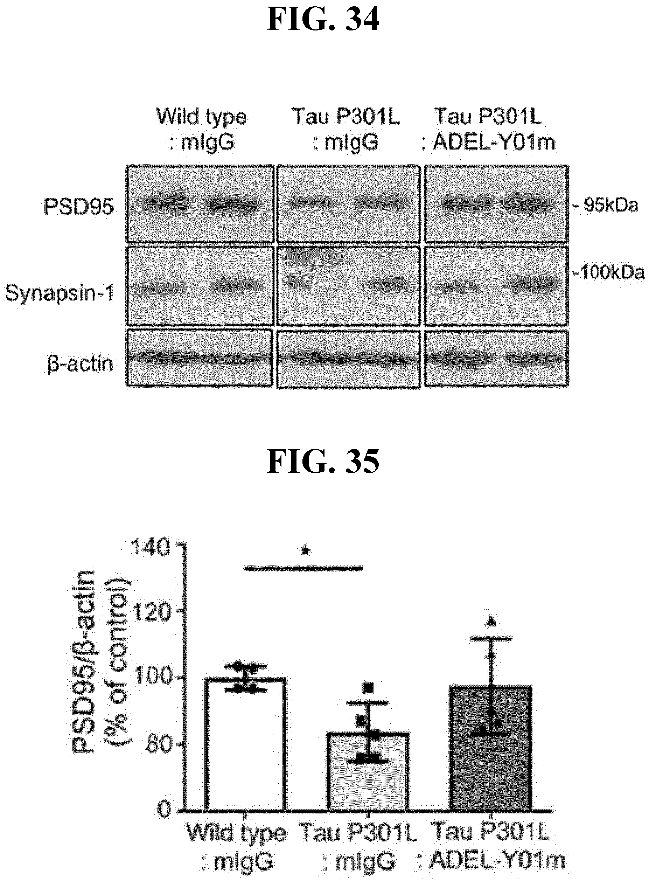

As a result, increased expression levels of PSD59 and synapsin-1 were observed in the brain tissues of Tau-P301L dementia mice, into which IgG had been intracerebroventricularly administered, as compared with the brain tissues of normal mice. On the other hand, decreased expression levels of PSD59 and synapsin-1 were observed in the brain tissues of Tau-P301L dementia mice, into which the ADEL-Y01m antibody had been intracerebroventricularly administered, as compared with the brain tissues of Tau-P301L dementia mice, into which IgG had been intracerebroventricularly administered (Figs. 34 to 36).

### Experimental Example 11. Identification of synapse-improving effect caused by ADEL-Y01m antibody: Intraperitoneal administration

In order to identify a synapse-improving effect following administration of the ADEL-Y01m antibody, brain tissues of the Tau-P301L dementia mice, into which IgG or ADEL-YOlm antibody had been intraperitoneally administered in an amount of 50 mg/kg, and the normal mice were extracted, and western blotting was performed on synapse-related proteins. Here, the western blotting was performed in the same manner as in Experimental Example 10.

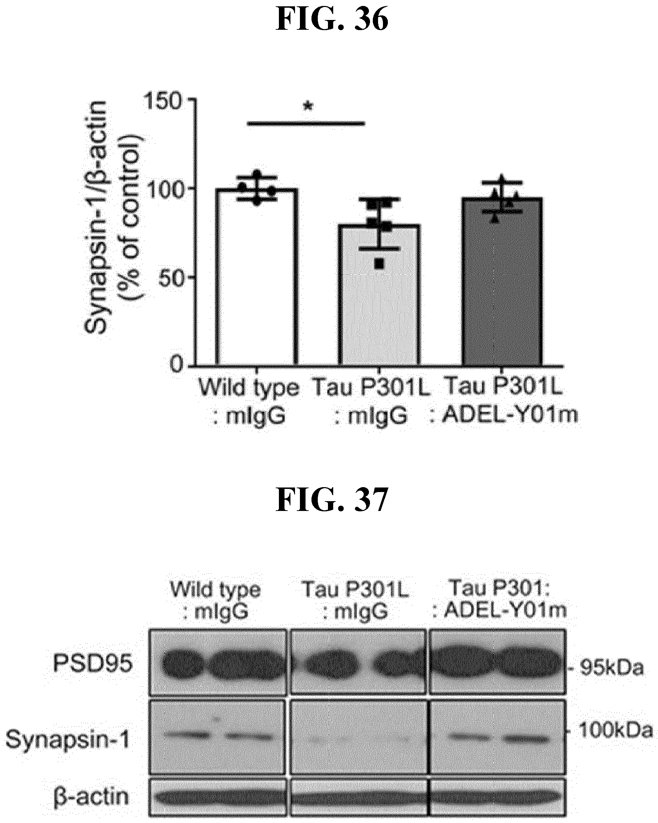

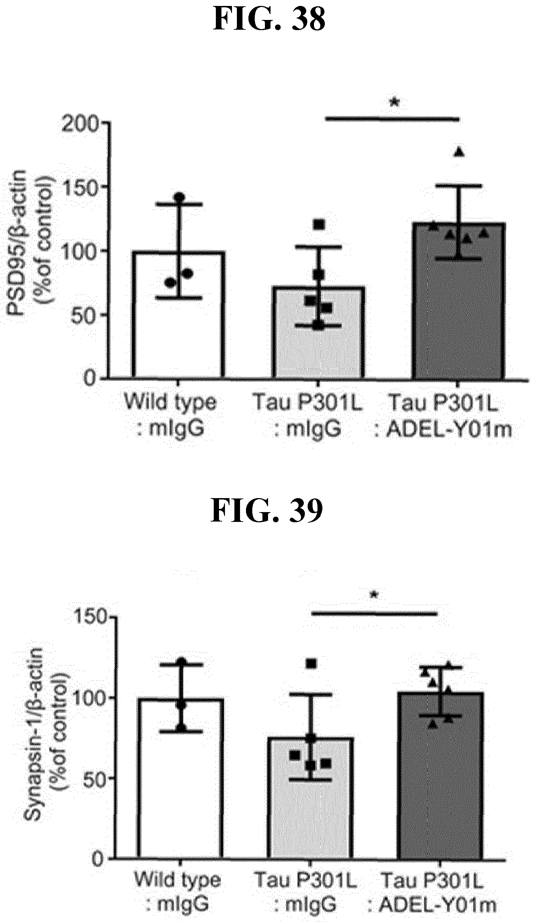

As a result, increased expression levels of PSD59 and synapsin-1 were observed in the brain tissues of Tau-P301L dementia mice, into which IgG had been intraperitoneally administered, as compared with the brain tissues of normal mice. On the other hand, decreased expression levels of PSD59 and synapsin-1 were observed in the brain tissues of Tau-P301L dementia mice, into which the ADEL-Y01m antibody had been intraperitoneally administered, as compared with the brain tissues of Tau-P301L dementia mice, into which IgG had been intraperitoneally administered (Figs. 37 to 39).

### Experimental Example 12. Identification of penetration of ADEL-Y01m antibody into brain tissue and binding thereof to antigen following intraperitoneal administration of ADEL-Y01m antibody

In order to identify antibody distribution in brain tissue after intraperitoneal administration of the ADEL-Y01m antibody, the Tau-P301L dementia mice, into which the ADEL-Y01m antibody had been intraperitoneally administered in an amount of 50 mg/kg, and the normal mice were subjected to cardiac perfusion with physiological saline. Then, brain tissues were extracted therefrom and subjected to immunoprecipitation with protein-G sepharose (PGS). Thereafter, western blotting was performed using an anti-mouse IgG antibody. Here, the western blotting was performed in the same manner as in Experimental Example 1, and anti-Tau5 antibody (Invitrogen, AHB0042), anti-acetyl-K280 antibody (Anaspec, AS-56077), and HRP-labeled anti-mouse IgG antibody (Bioxcell, BE0083) were used.

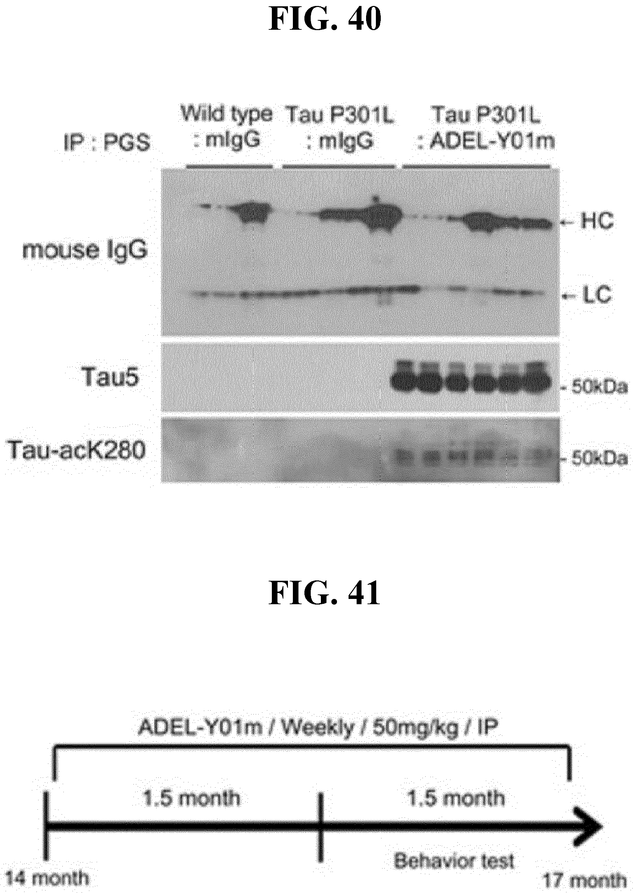

As a result, no antibody was detected in the brain tissues of the Tau-P301L dementia mice, into which IgG had been intraperitoneally administered. On the other hand, it was identified that the antibody was present in the brain tissues of the Tau-P301L dementia mice, into which the ADEL-Y01m antibody had been intraperitoneally administered (Fig. 40).

### Experimental Example 13. Identification of behavior-improving effect caused by ADEL-Y01m antibody in aged dementia mouse model: Intraperitoneal administration

In order to identify therapeutic efficacy of the ADEL-Y01m antibody, an aged dementia mouse model was produced using aged 14-month-old mice in the same manner as in Preparation Example 3. Using the aged dementia mouse model, the ADEL-Y01m antibodies prepared in Preparation Example 2 were administered thereto according to the experimental schedule illustrated in Fig. 41.

### Experimental Example 13.1. Identification of behavior-improving effect through nest building test

A nest building test which can identify a multimodal brain function of the mice was performed in the same manner as Experimental Example 4.1.

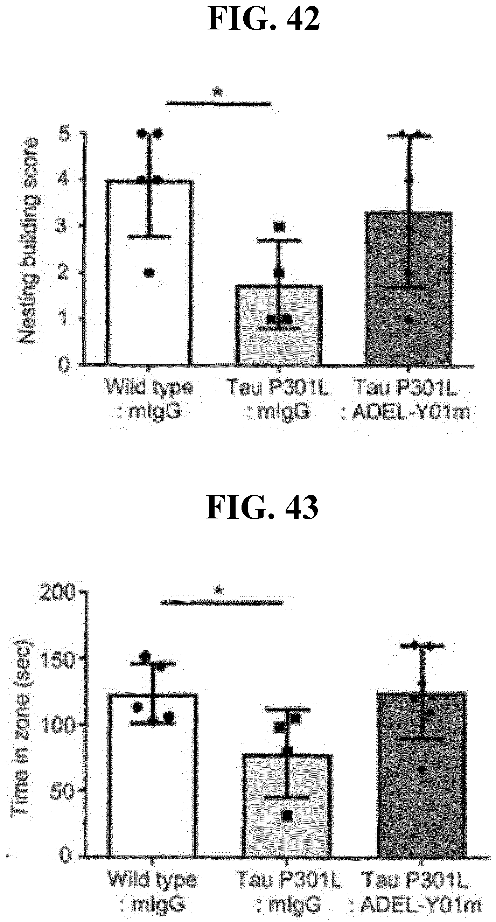

As a result, it was identified that the Tau-P301L dementia mice exhibit about 60% decrease in building score as compared with the normal mice and that the Tau-P301L-ADEL-Y01m dementia mice, into which the ADEL-Y01m antibody has been administered, exhibit a similar building score to the normal mice (Fig. 42).

### Experimental Example 13.2. Identification of behavior-improving effect through Y-maze test

A Y-maze test was performed, in the same manner as in Experimental Example 4.2, to identify restoration of cognitive function.

As a result, in a case of the Tau-P301L mice, the time spent staying in the A or C zone was around 80 seconds which was about 25% decreased as compared with the normal mice. On the other hand, in a case of the Tau-P301L-ADEL-Y01m dementia mice into which the ADEL-YOlm antibody had been administered, the time spent staying in the A or C zone was around 120 seconds which was similar to the normal mice. From these results, it was identified that the short-term memory capacity of the Tau-P301L-ADEL-Y01m dementia mice is restored (Fig. 43).

### Experimental Example 13.3. Identification of behavior-improving effect through water maze test

An underwater maze test was performed in the same manner as in Experiment 4.3.

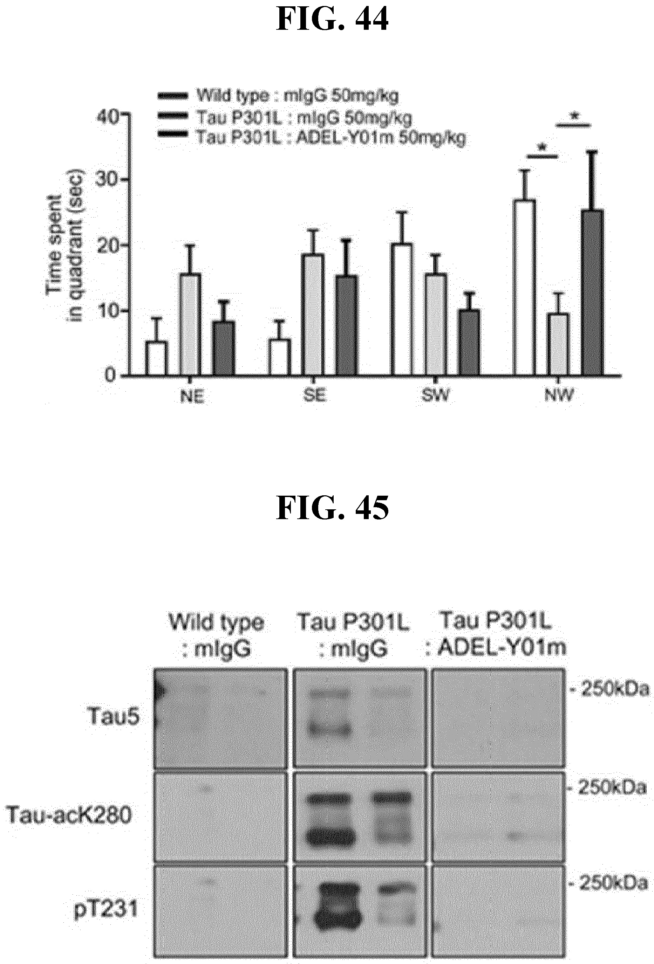

As a result, on the 5^{th} day, evaluation was performed on the time spent staying in the NW zone where the platform had been placed. As a result, it was identified that the Tau-P301L mice stay in the NW zone for 10 seconds or shorter, whereas the normal mice and the Tau-P301L-ADEL-Y01m dementia mice into which the ADEL-Y01m antibody has been administered stay in the NW zone for 25 seconds or longer (Fig. 44).

### Experimental Example 14. Identification of tauopathy-alleviating effect caused by ADEL-Y01m antibody in aged dementia mouse model: Intraperitoneal administration

In order to identify a tauopathy-alleviating effect following administration of the ADEL-Y01m antibody, brain tissues of the aged Tau-P301L dementia mice, into which IgG or ADEL-Y01m antibody had been intraperitoneally administered in an amount of 50 mg/kg, and the normal mice were extracted, and expression levels of the entire tau protein (Tau5), acetylated tau protein (Tau-acK280), and phosphorylated tau (pT231) were checked through western blotting. Here, the western blotting was performed in the same manner as in Experimental Example 1, and anti-Tau5 antibody (Invitrogen, AHB0042), anti-pT231 antibody (Thermo, MN1040), anti-acetyl-K280 antibody (Anaspec, AS-56077), and HRP-labeled anti-mouse IgG antibody (Bioxcell, BE0083) were used.

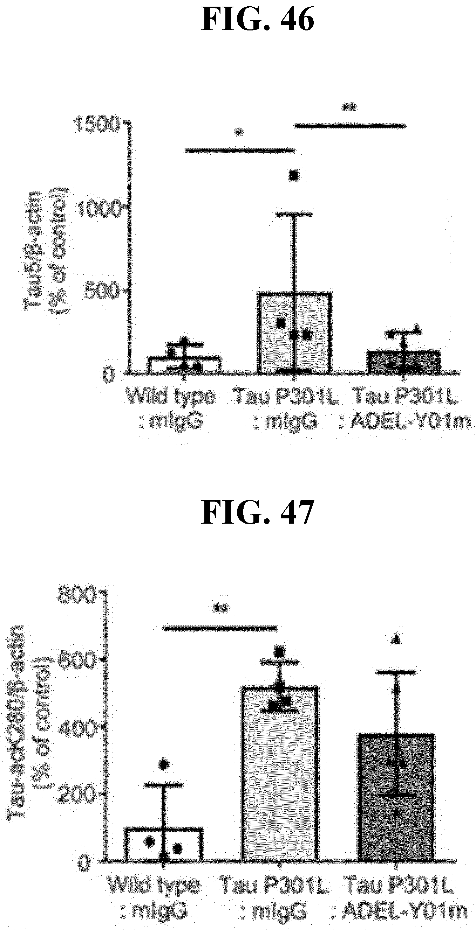

As a result, increased expression levels of an oligomeric form of the entire tau protein, phosphorylated tau protein, and acetylated tau protein were observed in the brain tissues of the Tau-P301L dementia mice, into which IgG had been intraperitoneally administered. On the other hand, remarkably decreased expression levels of the entire tau protein, the phosphorylated tau protein, and the acetylated tau protein were observed in the brain tissues of the Tau-P301L dementia mice, into which the ADEL-Y01m antibody had been intraperitoneally administered, as compared with the brain tissues of the Tau-P301L dementia mice, into which IgG had been intraperitoneally administered (Figs. 45 to 48).

In addition, brain tissues of the aged Tau-P301L dementia mice, into which IgG or ADEL-YOlm antibody had been intraperitoneally administered in an amount of 50 mg/kg, and the normal mice were extracted, and cerebral cortical tissues were partially isolated therefrom using formic acid. In the cerebral cortical tissues, insoluble tau aggregates were checked through semi-denaturating western blotting. Here, the semi-denaturating western blotting was performed in the same manner as in Experimental Example 9.

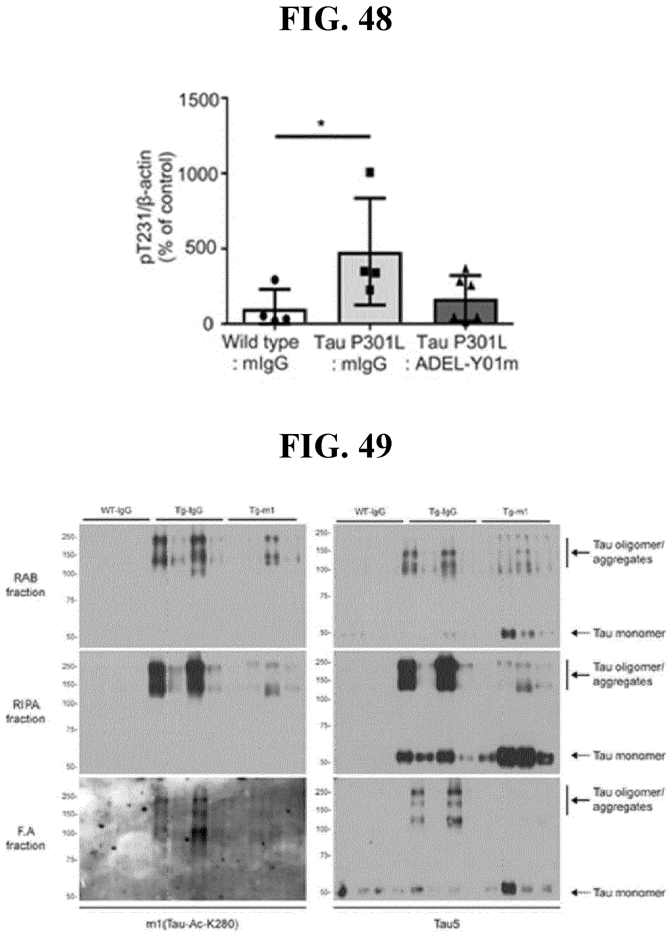

As a result, an increase in insoluble tau aggregates was observed in the brain tissues of the Tau-P301L dementia mice, into which IgG had been intraperitoneally administered. On the other hand, a remarkable decrease in insoluble tau aggregates was observed in the brain tissues of the Tau-P301L dementia mice, into which the ADEL-Y01m antibody had been intraperitoneally administered, as compared with the brain tissues of the Tau-P301L dementia mice, into which IgG had been intraperitoneally administered (Fig. 49).

### III. Identification of Tau seeding inhibitory effect of anti-tau antibody

### Experimental Example 15. Identification of tau aggregation and seeding of acetylated tau protein

First, the wild-type tau protein (Tau) was reacted *in vitro* with the P300 enzyme, an acetyltransferase. Cultured nerve cells were treated with acetylated tau protein (Tau-K280-ac), which had been reacted with the P300. In order to identify whether the acetylated tau protein induces intracellular aggregation, treatment with the acetylated tau protein or wild-type tau protein was performed, and western blotting was performed on the donor cell lysate using anti-HA antibody (Sigma-Aldrich, #11867423001) when 1 hour or 20 hours had elapsed.

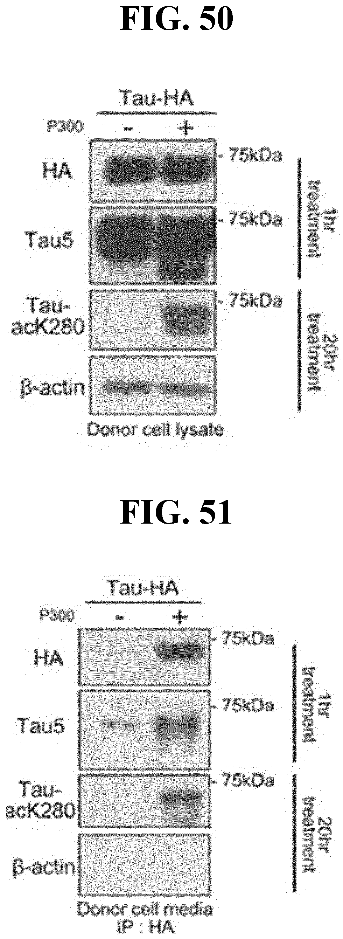

Here, the western blotting was performed in the same manner as in Experimental Example 1, and anti-Tau5 antibody (Invitrogen, AHB0042), anti-acetyl-K280 antibody (Anaspec, AS-56077), anti-β-actin antibody (Sigma, A5441), anti-HA antibody (Sigma, #11867423001), and HRP-labeled anti-mouse IgG antibody (Bioxcell, BE0083) were used. As a result, increased aggregation of the entire tau protein (Tau5) and the acetylated tau protein (Tau-acK280) was observed in the lysate of donor cells treated with the acetylated tau protein (Fig. 50).

In addition, in order to identify whether the amount of tau proteins secreted out of the cell is changed, the donor cell media were subjected to immunoprecipitation using anti-HA antibody (Sigma-Aldrich, #11867423001), and then western blotting was performed.

Specifically, the donor cell media were centrifuged at 13,000 rpm for 1 minute, to remove the cell debris. Then, the donor cell media were treated with protein G-agarose beads (GE Healthcare Life Sciences, Piscataway, NJ, USA) and allowed to react at 4°C for 1 hour. Subsequently, treatment with 1 µl of anti-HA antibody was performed and reaction was allowed to proceed at 4°C overnight. The next day, Protein G sepharose beads were added thereto and reaction was allowed to proceed at 4°C for 1 hour. Then, centrifugation was performed at 6,000 rpm for 1 minute at 4°C to collect samples. Each of the samples was washed twice with PBS containing 0.1% Triton X-100. The sample was treated with a sample buffer, and then western blotting was performed. Here, the western blotting was performed in the same manner as that performed for the donor cell lysate.

As a result, an increase in the amounts of the entire tau protein (Tau5) and the acetylated tau protein (Tau-acK280) was observed in the media of donor cells treated with the acetylated tau protein (Fig. 51).

In addition, other nerve cells (recipient cells) were treated with the donor cell media, and the recipient cell lysate was collected when 1 hour or 20 hours had elapsed. Western blotting was performed on each sample using the tau antibody. Here, the western blotting was performed in the same manner as in Experimental Example 1, and anti-Tau5 antibody (Invitrogen, AHB0042), anti-acetyl-K280 antibody (Anaspec, AS-56077), anti-β-actin antibody (Sigma, A5441), anti-HA antibody (Sigma, #11867423001), and HRP-labeled anti-mouse IgG antibody (Bioxcell, BE0083) were used.

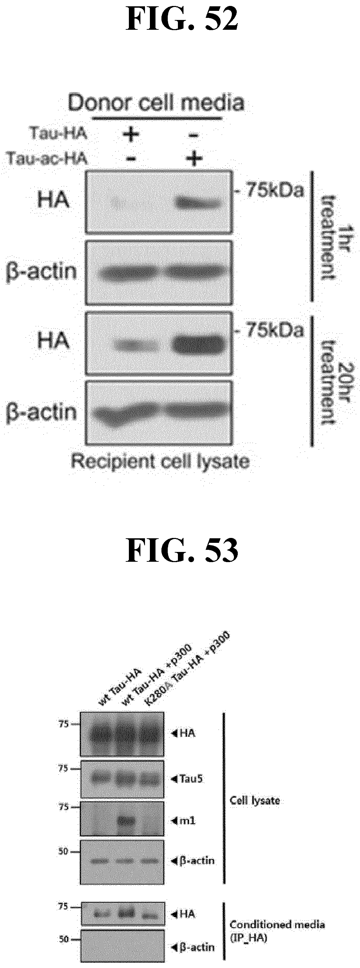

As a result, tau protein aggregates were observed in the lysate of recipient cells treated with the media of donor cells (Tau-ac-HA) treated with the acetylated tau protein (Figs. 52 and 53).

### Experimental Example 16. Identification of tau seeding inhibitory effect caused by ADEL-Y01h antibody: In vitro

In order to identify an *in vitro* tau seeding inhibitory effect caused by the ADEL-YO1h01 v01 antibody, K280-ac protein fragment (mAC), an aggregate thereof (aAC), or a Sarkosyl insoluble fraction in brain tissues of patients with Alzheimer's disease was administered into the tau-FRET stable cell system, and a level of tau seeding was measured. To tau-seeded cells were administered, respectively, IgG and ADEL-Y01h01_v01 antibody. Comparative analysis was performed therefor.

First, an aggregate of the K280-ac protein fragments to be used for treating cells was prepared as follows. The K280-ac protein fragments having a concentration of 25 µM were dissolved in a 25 mM DTT solution at a temperature of 24°C for 1 hour, and the resulting product was dissolved, with stirring, in a solution of (2-hydroxyethyl)-1-piperazine ethane sulfonic acid (HEPES, 10 mM, pH 7.4), NaCl (100 mM), and heparin (25 mM), using Eppendorf Thermomixer C under a condition of 700 rpm at a temperature of 37°C. By doing so, the aggregate of the K280-ac protein fragments was prepared.

Thereafter, HEK293 Tau RD P301S-FRET Biosensor (ATCC CRL-3275) was dispensed in a 96-well plate at 3.5 × 10⁴ cells per well. 18 hours later, it was identified whether about 60% of each well is coated with the HEK 293 Tau RD P301S-FRET Biosensor. Then, 8.75 µl of OPTI-MEM, 1.25 µl of Lipofectamine 2000 (Invitrogen), and the K280-ac protein fragment, the aggregate thereof, or the Sarkosyl insoluble fraction were mixed, and the mixture was allowed to react at room temperature for 20 minutes. Thereafter, each well was subjected to treatment with the mixture, and then incubation was performed at a temperature of 37°C for 24 hours. Then, fluorescence wavelength analysis was performed using the CLARIOstar^{®} (BMG Labtech) instrument.

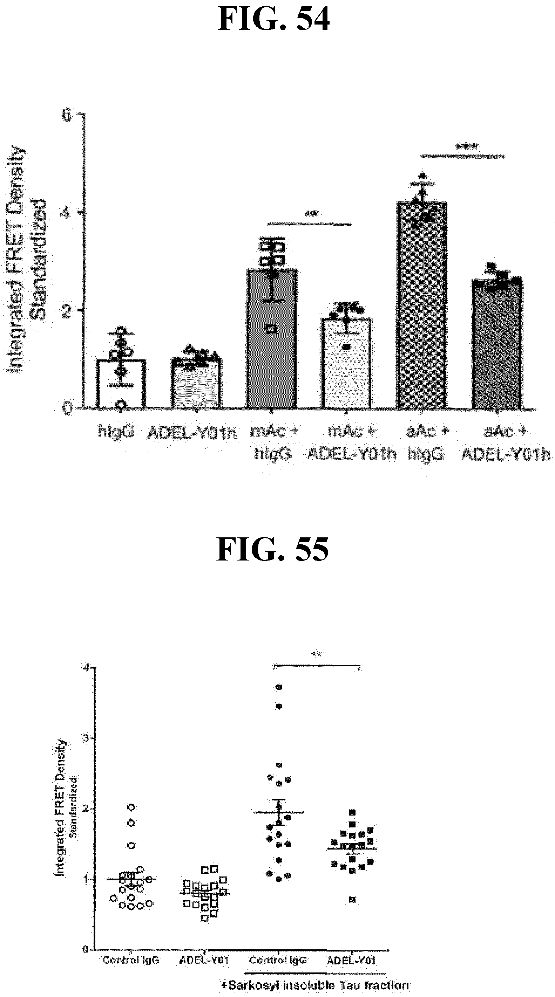

As a result, in a case where the ADEL-Y01h01_v01 antibody was administered, the cells treated with the K280-ac protein fragment exhibited a decreased integrated FRET density; and in a case where the ADEL_v01 antibody was administered, the cells treated with the aggregate of the K280-ac protein fragments also exhibited a decreased integrated FRET density (Fig. 54). In addition, in a case where the ADEL-Y01h antibody was administered, the cells treated with the Sarkosyl insoluble fraction in brain tissues of patients with Alzheimer's disease exhibited a decreased integrated FRET density (Fig. 55). From these results, it was identified that in a case where the ADEL-Y01h01_v01 antibody was administered, tau seeding was inhibited.

### Experimental Example 17. Identification of tau seeding inhibitory effect caused by ADEL-Y01m antibody: In vivo

In order to identify an *in vivo* tau seeding inhibitory effect caused by the ADEL-Y01m antibody, IgG or ADEL-Y0 1m antibody had been intravenously injected starting from 2 weeks before the Sarkosyl insoluble fraction from patients with Alzheimer's disease was administered to CA1 layer in the left hippocampus of the Tau-P301L dementia mice produced in Preparation Example 3. The Sarkosyl insoluble fraction was administered to the CA1 layer in the left hippocampus of the Tau-P301L dementia mice. Subsequently, brain tissue was extracted at the expense of each mouse, and then sectioned frozen. The brain sections were subjected to immunohistological staining with an anti-AT8 antibody, and a level of tau seeding was checked.

Specifically, a Sarkosyl insoluble fragment (3 µl, 3.3 µg/µl) in brain tissue from a patient with Alzheimer's disease at Braak stage IV was subjected to sonication and injected into CA1 layer (anterior-posterior: -1.9 mm, inside-outside: 1.5 mm, left: -1.8 mm) in the left hippocampus of 6-month-old Tau-P301L dementia mice. The Tau-P301L dementia mice had been intravenously injected with IgG or ADEL-Y01m antibody in an amount of 20 mg/kg/week starting from 2 weeks before administration of the Sarkosyl insoluble fragment; and the mice were intravenously administered IgG or ADEL-Y01m antibody once a week from the week of administration of the Sarkosyl insoluble fragment to Week 12. Here, the Sarkosyl insoluble fragment was injected at a rate of 0.2 µl/min using a 10 µl glass syringe (Hamilton, 0.49 mm, Reno, NV), and the needle was removed after 5 minutes of waiting before removal in order to prevent backflow. After 12 weeks, brain tissue was extracted at the expense of each mouse and cut into 30 µm thick frozen sections using Leica CM1860.

For immunohistological staining, each of the brain sections was washed with Buffer A (1% NGS, 0.2% Triton X-100 and 30% H₂O₂, and PBS). The brain section was subjected to treatment with PBS containing 1% NGS and 0.2% Triton X-100, and IgG or ADEL-Y01m antibody, and allowed to react at 4°C overnight. The brain section was washed several times with PBS solution, and then reacted with a secondary antibody (Vector Laboratories) at room temperature for 1 hour. Thereafter, the brain section was subjected to immunostaining using the Vectastain ABC kit (Vector Laboratories), and the immunostaining was performed according to the manufacturer's manual. The stained brain sections were analyzed using the ImageJ software (NIH, Bethesda, MD), and three brain sections containing the hippocampus were quantified. For nerve cells stained with the anti-AT8 antibody, measurement was performed by three blind testers with the brain sections used in the experiment, and quantitative analysis was performed at specific threshold levels. Three brain sections between Bregma -1.75 and Bregma -2.25 in 7 Tau-P301L dementia mice, into which IgG had been administered, and 8 Tau-P301L dementia mice, into which the ADEL-Y01m antibody had been administered, were used for analysis. A t-test was used for statistical analysis.

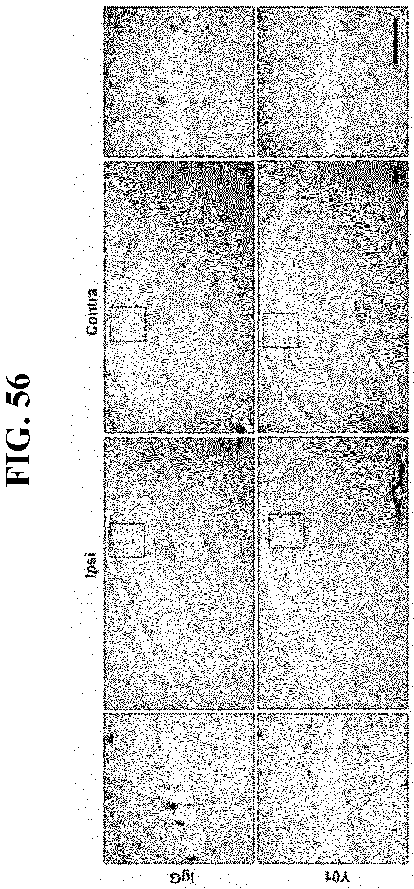

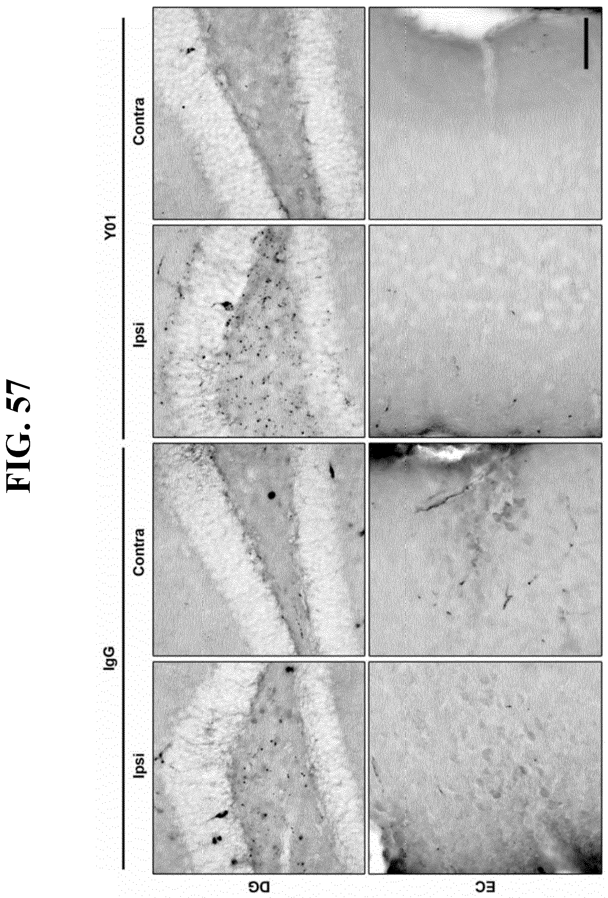

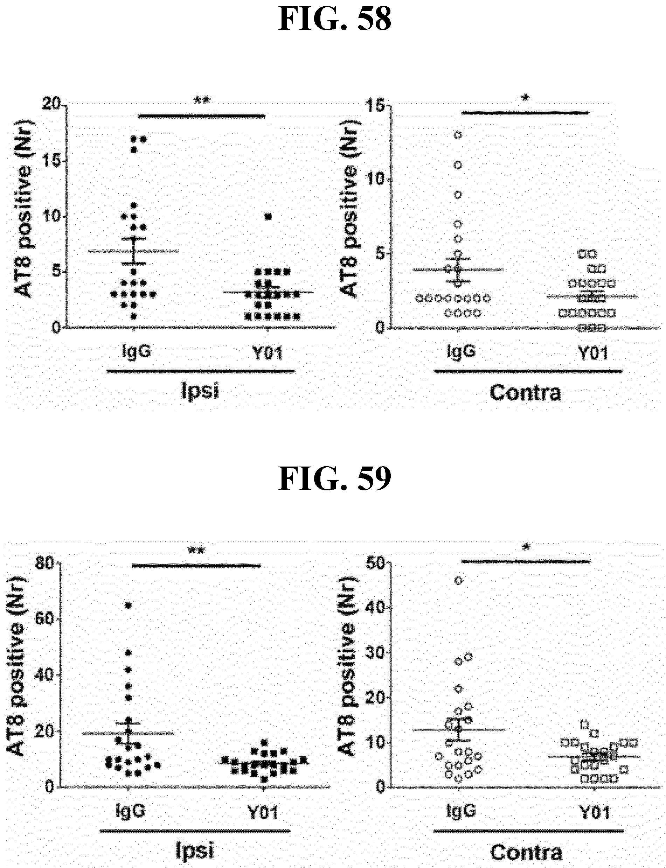

As a result, for the Tau-P301L dementia mice, into which IgG had been administered, staining of nerve cell bodies was strongly observed in brain regions (cortex, hippocampus) opposite to the side where the patient's insoluble fragment had been administered. On the other hand, for the Tau-P301L dementia mice, into which the ADEL-Y01m antibody had been administered, decreased staining was observed in the hippocampus and cortex (Figs. 56 to 59).

### Experimental Example 18. Identification of tau aggregation and seeding inhibitory effects caused by ADEL-Y01m/ADEL-Y01h antibody

In order to identify a tau aggregation inhibitory effect caused by the ADEL-YOlm antibody, an *in vitro* tau aggregation assay was performed. K18 protein fragment produced *in vitro* was acetylated by being reacted with 1.5 µg of P300 and 1 mM Acetyl-CoA at a temperature of 30°C for 3 hours. Thereafter, monomeric K18 protein fragment was reacted with 25 µM acetylated K18 protein fragment (K18-P301L) at room temperature for 1 hour. Then, the resultant was treated with 25 mM heparin 57, HEPES (10 mM, pH 7.4), and NaCl (100 mM), and stirred at a condition of 700 rpm to induce aggregation of the tau proteins. Then, tau aggregation and seeding inhibitory effects thereof were analyzed, using the ADEL-Y01m antibody, through western blotting and thioflavin-T assay. It was identified by thioflavin-T assay that aggregation of acetylated tau proteins is inhibited in a case where treatment with the ADEL-Y01h antibody is performed, as compared with a case where the acetylated tau proteins are aggregated.

First, for fibrillation reaction, 10 M to 20 M acetylated tau protein (2N4R) was incubated at 37°C in a solution obtained by adding heparin (Sigma-Aldrich) and DTT at a concentration of 2 mM in 100 mM sodium acetate buffer (pH 7.0). The protein and ThT solution (at a ratio of 1:1) was added to each well. ThT fluorescence analysis at different time points was measured using CLARIOstar^{®} (BMG Labtech, Ortenberg, Germany) at 450 nm (excitation) and 510 nm (emission) wavelengths.

Cultured mouse nerve cells were treated with the aggregate of acetylated proteins, and western blotting was performed to identify the results.

For the experiment, primary cerebral cortical neurons (DIV10) were treated with monomeric K18-P301L, aggregated K18-P301L, or acetylated K18-P301L at a concentration of 3 µg/ml for 24 hours. The neurons were pretreated with the ADEL-YOlm antibody at 3 µg/ml for 30 minutes prior to the K18 protein treatment.

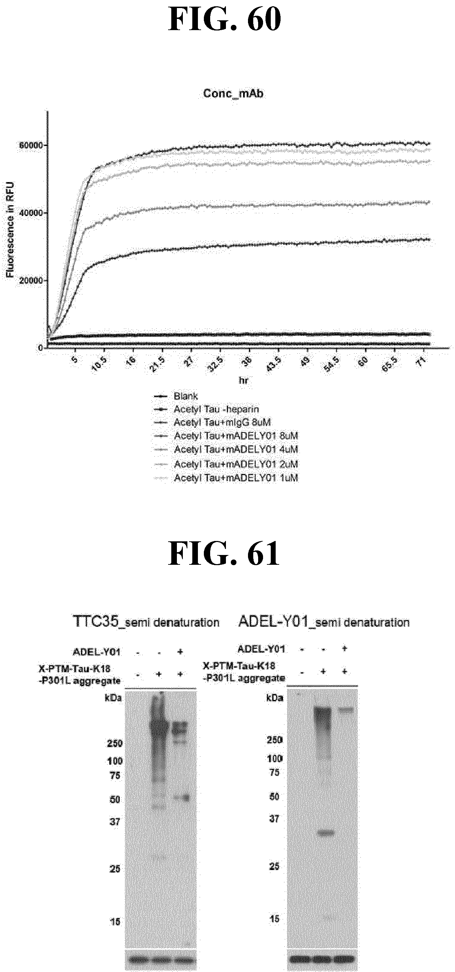

As a result, it was seen that many tau aggregates enter the nerve cells and induce aggregation of intrinsic tau proteins; however, it was identified that in a case of being treated simultaneously with the ADEL-Y01m antibody, such phenomena are decreased (Figs. 60 and 61).

### Experimental Example 19. Identification of nerve cell protective effect caused by ADEL-Y01m antibody

In order to identify a nerve cell protective effect caused by the ADEL-Y01m antibody antibody, cerebral cortical nerve cells were first isolated from the mouse brain during embryonic days. Specifically, brain tissue was extracted at the expense of a 16-day-old mouse, and the cerebral cortex was isolated therefrom. Thereafter, the cerebral cortical tissue was dissected in calcium- and magnesium-free Hank's balanced salt solution (HBSS) and incubated in a 0.125% trypsin solution at 37°C for 15 minutes. Trypsin was inactivated by treatment with Dulbecco's modified Eagle's medium (DMEM) containing 20% fetal bovine serum, and the cerebral cortical tissue was pulverized using a pipette. The resulting cell suspension was diluted in NB medium (neurobasal medium) supplemented with B27 supplement (GibcoBRL), and dispensed onto a plate coated with poly-D-lysine (Sigma) at 50 µg/ml and laminin (GibcoBRL) at 1 µg/ml. The nerve cells were cultured at a condition of 37°C and 5% CO₂ for 10 days. Then, the cultured nerve cells were treated with an aggregate of acetylated K18 protein fragments at 3 µg/ml and IgG or ADEL-Y01m antibody, and allowed to react for 24 hours.

After 24 hours, the supernatant was collected and analyzed using a cytotoxicity detection kit (LDH, Roche Applied Sciences). As a colorimetric assay for quantifying cell death, a method of measuring activity of lactate dehydrogenase (LDH), which was released into the supernatant from the cytosol of damaged nerve cells, was used. Absorbance at a wavelength of 490 nm was measured using the Tecan Infinite^{®} 200 instrument. The results were expressed in percentage of cell viability as compared with a negative control (Blank).

In addition, the cell viability was measured using MTT (Thiazolyl Blue Tetrazolium Bromide, Sigma-Aldrich, St. Louis, MO, USA) reduction assay. Absorbance at a wavelength of 540 nm was measured using the TecanInfinite^{®}200 instrument, and the results were expressed in percentage as compared with a negative control (Blank).

In addition, the results were obtained under a condition of two independent cultures and 6 wells, and all values were expressed as mean ± s.e. of the mean. One-way ANOVA with Tukey's multiple comparison test was used for statistical analysis.

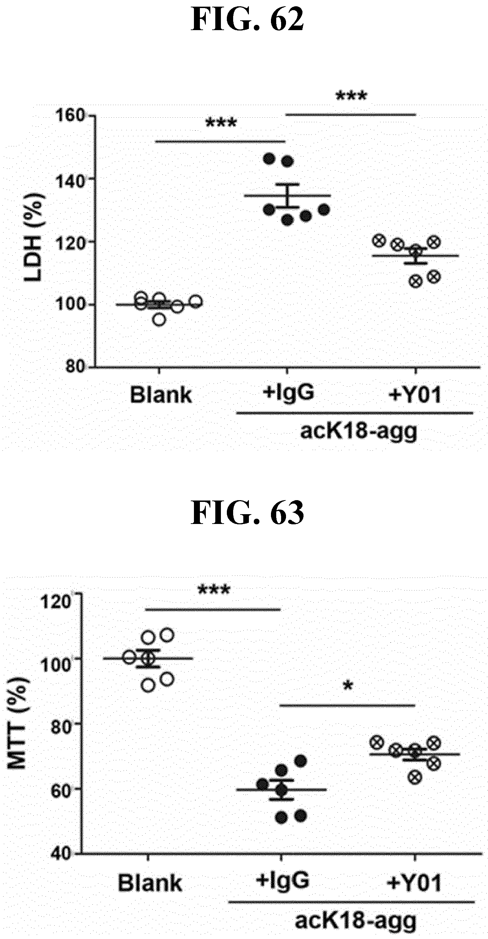

As a result, as illustrated in Fig. 62, high LDH activity was observed in the IgG-treated nerve cells. On the other hand, decreased LDH activity was observed in the ADEL-Y01m antibody-treated nerve cells as compared with the IgG-treated nerve cells. In addition, as illustrated in Fig. 63, greatly decreased viability was observed in the IgG-treated nerve cells. On the other hand, significantly increased viability was observed in the ADEL-Y01m antibody-treated nerve cells as compared with the IgG-treated nerve cells. From these results, it was identified that the ADEL-Y01m antibody exhibited a protective effect against damages in nerve cells caused by aggregation of acetylated tau proteins.

### Experimental Example 20. Identification of microglial phagocytosis promotion effect caused by ADEL-Y01m antibody

In order to identify a microglial phagocytosis promotion effect caused by the ADEL-Y01m antibody, primary microglia were first isolated from the cerebral cortex of 2- to 3-day-old mice and cultured according to the method described in McDonald DR et al., J Neurosci. 1997; 17: 2284-94.

Thereafter, for analysis of the microglial phagocytosis, the acetylated K18 protein fragment was labeled with HiLyte^{™} Fluor 488 according to the manufacturer's instructions. For aggregation of HiLyte^{™} Fluor 488-labeled acetylated K18 proteins, the proteins were respectively incubated at 37°C in 100 mM sodium acetate buffer (pH 7.0) containing heparin (Sigma-Aldrich, St. Louis, MO, USA) and 2 mM DTT so that aggregation thereof was induced.

The microglia were dispensed in a 12-well-plate at 5×10⁴ cells/well, and then the aggregate of acetylated K18 protein fragments (3 µg/ml) was allowed to react at 37°C for 2 hours. Pretreatment with IgG or the ADEL-Y01m antibody at a concentration of 4.5 µg/ml was performed 30 minutes before treatment with the aggregate of acetylated K18 protein fragments. For analysis of phagocytosis, the cells were separated by treatment with trypsin, and then fixed with 1% paraformaldehyde (PFA). For flow cytometry, treatment with an HBSS solution containing 200 µl of 2% fetal bovine serum was performed and analysis was performed using the FACSCanto^{™} II (BD Biosciences) instrument and 488 fluorescence filter.

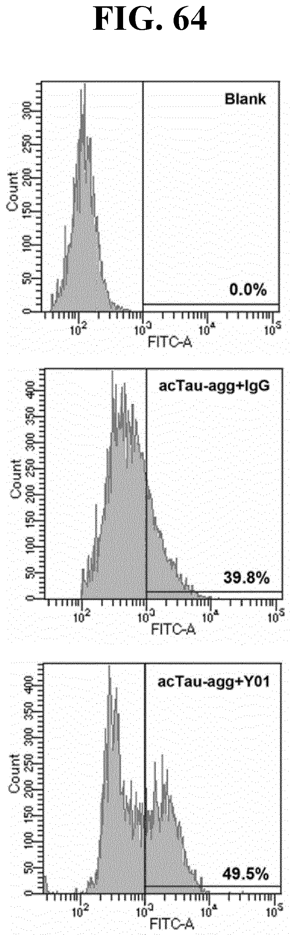

As a result, promoted phagocytosis was observed in the ADEL-Y01m antibody-treated microglia as compared with the IgG-treated microglia (Fig. 64).

### Statistical analysis

Statistical analysis was performed using GraphPad software (GraphPad Prism v7.0 and GraphPad Prism v8.0, GraphPad Software, San Diego, CA, USA). Data were analyzed by one-way ANOVA (Tukey's post hoc test) and Student's t-test. P values less than 0.05 were considered significant.

### [Accession Number]

Name of Depository Organization: Korean Collection for Type Cultures (KCTC)
Accession Number: KCTC14155BP
Accession Date: March 18, 2020

## Claims

1. An anti-tau antibody or an antigen-binding fragment thereof, comprising:
a heavy chain variable region (VH) that includes:
a heavy chain CDR1 having the amino acid sequence represented by SEQ ID NO: 1;
a heavy chain CDR2 having the amino acid sequence represented by SEQ ID NO: 2; and
a heavy chain CDR3 having the amino acid sequence represented by SEQ ID NO: 3, and
a light chain variable region (VL) that includes:
a light chain CDR1 having the amino acid sequence represented by SEQ ID NO: 4;
a light chain CDR2 having the amino acid sequence represented by SEQ ID NO: 5; and
a light chain CDR3 having the amino acid sequence represented by SEQ ID NO: 6.

2. The anti-tau antibody or antigen-binding fragment thereof of claim 1, wherein the heavy chain variable region includes any one amino acid sequence selected from the group consisting of SEQ ID NOs: 7 to 11, and the light chain variable region includes any one amino acid sequence selected from the group consisting of SEQ ID NOs: 12 to 15.

3. The anti-tau antibody or antigen-binding fragment thereof of claim 1, wherein the antibody or the antigen-binding fragment thereof is any one selected from the group consisting of:
an anti-tau antibody or antigen-binding fragment thereof which comprises a heavy chain variable region having the amino acid sequence represented by SEQ ID NO: 7 and a light chain variable region having the amino acid sequence represented by SEQ ID NO: 12;
an anti-tau antibody or antigen-binding fragment thereof which comprises a heavy chain variable region having the amino acid sequence represented by SEQ ID NO: 8 and a light chain variable region having the amino acid sequence represented by SEQ ID NO: 13;
an anti-tau antibody or antigen-binding fragment thereof which comprises a heavy chain variable region having the amino acid sequence represented by SEQ ID NO: 8 and a light chain variable region having the amino acid sequence represented by SEQ ID NO: 14;
an anti-tau antibody or antigen-binding fragment thereof which comprises a heavy chain variable region having the amino acid sequence represented by SEQ ID NO: 8 and a light chain variable region having the amino acid sequence represented by SEQ ID NO: 15;
an anti-tau antibody or antigen-binding fragment thereof which comprises a heavy chain variable region having the amino acid sequence represented by SEQ ID NO: 9 and a light chain variable region having the amino acid sequence represented by SEQ ID NO: 13;
an anti-tau antibody or antigen-binding fragment thereof which comprises a heavy chain variable region having the amino acid sequence represented by SEQ ID NO: 9 and a light chain variable region having the amino acid sequence represented by SEQ ID NO: 14;
an anti-tau antibody or antigen-binding fragment thereof which comprises a heavy chain variable region having the amino acid sequence represented by SEQ ID NO: 9 and a light chain variable region having the amino acid sequence represented by SEQ ID NO: 15;
an anti-tau antibody or antigen-binding fragment thereof which comprises a heavy chain variable region having the amino acid sequence represented by SEQ ID NO: 10 and a light chain variable region having the amino acid sequence represented by SEQ ID NO: 13;
an anti-tau antibody or antigen-binding fragment thereof which comprises a heavy chain variable region having the amino acid sequence represented by SEQ ID NO: 10 and a light chain variable region having the amino acid sequence represented by SEQ ID NO: 14;
an anti-tau antibody or antigen-binding fragment thereof which comprises a heavy chain variable region having the amino acid sequence represented by SEQ ID NO: 10 and a light chain variable region having the amino acid sequence represented by SEQ ID NO: 15;
an anti-tau antibody or antigen-binding fragment thereof which comprises a heavy chain variable region having the amino acid sequence represented by SEQ ID NO: 11 and a light chain variable region having the amino acid sequence represented by SEQ ID NO: 13;
an anti-tau antibody or antigen-binding fragment thereof which comprises a heavy chain variable region having the amino acid sequence represented by SEQ ID NO: 11 and a light chain variable region having the amino acid sequence represented by SEQ ID NO: 14; and
an anti-tau antibody or antigen-binding fragment thereof which comprises a heavy chain variable region having the amino acid sequence represented by SEQ ID NO: 11 and a light chain variable region having the amino acid sequence represented by SEQ ID NO: 15.

4. The anti-tau antibody or antigen-binding fragment thereof of claim 1, wherein the fragment is any one selected from the group consisting of Fab, scFv, F(ab')₂, and Fv.

5. A polynucleotide encoding the anti-tau antibody or antigen-binding fragment thereof of claim 1.

6. An expression vector comprising the polynucleotide of claim 5.

7. A host cell comprising the expression vector of claim 6.

8. A method for producing the anti-tau antibody or antigen-binding fragment thereof of claim 1, the method comprising a step of culturing the host cell of claim 7.

9. A polynucleotide encoding a heavy or light chain of an antibody that includes the heavy chain variable region and the light chain variable region as described in claim 2.

10. A hybridoma having accession number KCTC 14155BP.

11. A pharmaceutical composition for preventing or treating a degenerative neurological disease, the pharmaceutical composition comprising as an active ingredient the anti-tau antibody or antigen-binding fragment thereof of claim 1.

12. The pharmaceutical composition of claim 11, wherein the degenerative neurological disease is a tau protein-mediated neurological disease.

13. The pharmaceutical composition of claim 12, wherein the tau protein-mediated neurological disease is tauopathy, primary age-related tauopathy, chronic traumatic encephalopathy, progressive supranuclear palsy, corticobasal degeneration, frontotemporal dementia, Lytico-Bodig disease, Parkinsonism, subacute sclerosing meningitis, lead encephalopathy, tuberous sclerosis, ganglioglioma, gangliocytoma, meningioangiomatosis, subacute sclerosing panencephalitis, Hallervorden-Spatz disease, or lipofuscinosis.

14. The pharmaceutical composition of claim 13, wherein the tauopathy is Alzheimer's disease (AD), progressive supranuclear palsy (PSP), corticobasal degeneration (CBD), Pick's disease (PiD), a group of related disorders collectively termed frontotemporal dementia with Parkinsonism linked to chromosome 17 (FTDP-17), amyotrophic lateral sclerosis (ALS), Creutzfeld-Jakob disease (CJD), dementia pugilitica (DP), Gerstmann-Straussler-Scheinker disease (GSSD), Lewy body disease, chronic traumatic encephalopathy (CTE), or Huntington's disease.

15. The pharmaceutical composition of claim 11, wherein the pharmaceutical composition is administered through any one route of administration selected from the group consisting of intracerebral, intracerebroventricular, intraperitoneal, percutaneous, intramuscular, intradural, intravenous, subcutaneous, and nasal routes of administration.

16. A composition for diagnosing a degenerative neurological disease, the composition comprising the anti-tau antibody or antigen-binding fragment thereof of claim 1.

17. A kit for preventing, treating, or diagnosing a degenerative neurological disease, the kit comprising the antibody or antigen-binding fragment thereof of claim 1, the polynucleotide of claim 5, the expression vector of claim 6, or the host cell of claim 7.

18. A use of the antibody or antigen-binding fragment thereof of claim 1 for preventing or treating a degenerative neurological disease.

19. A use of the antibody or antigen-binding fragment thereof of claim 1 for preparing a medicament that is used to prevent or treat a degenerative neurological disease.

20. A method for preventing or treating a degenerative neurological disease, the method comprising administering the anti-tau antibody or antigen-binding fragment thereof of claim 1 to a subject that is expected to have or currently has the degenerative neurological disease.
